(19) Europäisches Patentamt / European Patent Office / Office européen des brevets

(11) **EP 4 252 351 B1**

(12) **EUROPEAN PATENT SPECIFICATION**

(45) Date of publication and mention of the grant of the patent:
**04.03.2026  Bulletin 2026/10**

(21) Application number: **21806878.1**

(22) Date of filing: **05.10.2021**

(51) International Patent Classification (IPC):
*H03M 7/30* (2006.01)          *H03M 13/39* (2006.01)
*H03M 13/43* (2006.01)        *H03M 7/00* (2006.01)
*G06N 3/12* (2023.01)

(52) Cooperative Patent Classification (CPC):
**H03M 7/6041; G06N 7/01; H03M 7/6047;
H03M 13/63;** H03M 7/001; H03M 13/1102;
H03M 13/1515

(86) International application number:
**PCT/US2021/053462**

(87) International publication number:
**WO 2022/115159 (02.06.2022 Gazette 2022/22)**

(54) **TRELLIS BASED RECONSTRUCTION ALGORITHMS AND INNER CODES FOR DNA DATA STORAGE**

TRELLISBASIERTE REKONSTRUKTIONSALGORITHMEN UND INNERE CODES FÜR DNA-DATENSPEICHERUNG

ALGORITHMES DE RECONSTRUCTION À BASE DE TREILLIS ET CODES INTERNES POUR LE STOCKAGE DE DONNÉES D'ADN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **24.11.2020   US 202017102972**

(43) Date of publication of application:
**04.10.2023   Bulletin 2023/40**

(73) Proprietor: **Microsoft Technology Licensing, LLC
Redmond, WA 98052-6399 (US)**

(72) Inventors:
• **YEKHANIN, Sergey Mikhailovich
  Redmond, Washington 98052-6399 (US)**
• **GOPI, Sivakanth
  Redmond, Washington 98052-6399 (US)**
• **PFISTER, Henry David
  Redmond, Washington 98052-6399 (US)**
• **SRINIVASAVARADHAN, Sundara Rajan
  Redmond, Washington 98052-6399 (US)**

(74) Representative: **Goddar, Heinz J.
Boehmert & Boehmert
Anwaltspartnerschaft mbB
Pettenkoferstrasse 22
80336 München (DE)**

(56) References cited:
• **LENZ ANDREAS ET AL: "Concatenated Codes for Recovery From Multiple Reads of DNA Sequences", 2020 IEEE INFORMATION THEORY WORKSHOP (ITW), 29 October 2020 (2020-10-29), pages 1 - 5, XP055878128, ISBN: 978-1-7281-5962-1, Retrieved from the Internet <URL:https://arxiv.org/pdf/2010.15461> [retrieved on 20220112], DOI: 10.1109/ ITW46852.2021.9457675**
• **SRINIVASAVARADHAN SUNDARA RAJAN ET AL: "Algorithms for Reconstruction Over Single and Multiple Deletion Channels", ARXIV.ORG, 29 May 2020 (2020-05-29), pages 3389 - 3410, XP055878513, Retrieved from the Internet <URL:https://arxiv.org/pdf/2005.14388> [retrieved on 20220113], DOI: 10.1109/ TIT.2020.3033513**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **SAKOGAWA RYO ET AL: "Symbolwise MAP Estimation for Multiple-Trace Insertion/Deletion/ Substitution Channels", 2020 IEEE INTERNATIONAL SYMPOSIUM ON INFORMATION THEORY (ISIT), IEEE, 21 June 2020 (2020-06-21), pages 781 - 785, XP033814046, DOI: 10.1109/ ISIT44484.2020.9174050**

**Description**

BACKGROUND

Recently, there has been a significant increase in both the amount of data that needs to be stored as well as the different types of storage media that are used. Examples of such storage media include CDs, hard drives, solid state drives, tape, and so forth. The longevity of these media types, however, is quite limited. Indeed, these media types often last only a few years or a few decades. Furthermore, these media types are limited in their storage capacities.

In recent years, computer scientists have proposed using Deoxyribonucleic acid (DNA) as a new type of storage medium. DNA is a type of molecule that stores genetic information about a living organism. DNA is formed from four different nucleotide bases, namely: Adenine (A), Cytosine (C), Guanine (G), and Thymine (T). In this sense, DNA molecules effectively use a four-letter alphabet (A-C-G-T) to store information. As used herein, the terms "nucleotide bases," "nucleotides," or simply "bases" are interchangeable.

Due to the structure of DNA, DNA is able to store information for extremely long periods of time (e.g., thousands of years) and is able to store data in a highly compact manner (e.g., petabytes per gram of DNA). There are various steps that are performed in order to store and recover binary data on a DNA molecule, and these steps are referred to as a "DNA storage model." The steps in the model include an encoding process, a synthesis process, a storage process, a sequencing process, and a decoding process. Briefly, the encoding process involves segmenting a data file into binary code and then encoding the resulting binary data into short DNA sequences (called oligonucleotides) comprising various combinations of the A-C-G-T bases. Typically, DNA sequences used for data storage are no longer than 100-300 bases in length. Many copies of each DNA sequence are then generated during synthesis. The resulting DNA strands can then be stored as a dust, powder, liquid, and so on for almost an unlimited amount of time. Sequencing involves randomly sampling the DNA strands and identifying the sequences of bases in each strand. Decoding involves mapping the base sequence to binary data.

Each of these processes introduces noise and other corruptions into the sequences of bases. Examples of such noise and corruptions include nucleotide substitutions, insertions, and deletions. By way of example, during the synthesis process in which a new nucleotide base is added to a progressively growing DNA strand, a "substitution" occurs when a different base (i.e. one other than the desired one) is added to the strand; a "deletion" occurs when a base is not positioned where it should be positioned in the strand; and an "insertion" occurs when a base is positioned where it should not be positioned in the strand. Substitutions, insertions, and deletions can occur throughout any point of the model used to encode, synthesize, store, sequence, and decode.

In an effort to mitigate the effects of insertions, deletions, and substitutions (i.e. IDS errors), various solutions have been proposed focusing on the use of so-called "outer codes" and "inner codes." An outer code refers to a technique for recovering lost or undersampled DNA sequences by synthesizing redundant DNA sequences (i.e. DNA sequences that do not directly represent raw data but that instead are defined using error-correcting codes, such as LDPC codes or Reed Solomon codes). An inner code refers to a technique for detecting and correcting errors (e.g., noise and corruptions) within a single DNA strand by introducing redundant bases (e.g., by duplicating each base) within a single DNA strand. For example, perhaps a particular portion of data requires only 100 nucleotide bases, but the strand can hold about 150 bases. The other 50 units in the strand can be used to redundantly store portions of the 100 nucleotides already included in the strand.

Accordingly, using DNA strands as a storage medium is an emerging avenue of research, with DNA based storage systems promising high storage densities and long-term stability. A core problem arising in such systems is the design of error-correction codes and respective encoding and decoding algorithms for the DNA storage model. Document by Lenz Andreas ET AL: "Concatenated Codes for Recovery From Multiple Reads of DNA Sequences",2020 IEEE Information Theory Workshop (ITW), 29 October 2020, pages 1-5, 8,DOI: 10.1109/ITW46852.2021.9457675, describes one of those systems. Although the use of outer and inner codes has helped mitigate errors in DNA data storage systems, there is still a substantial need to further reduce error occurrences in DNA data storage.

The invention is defined by the appended claims.

BRIEF SUMMARY

Embodiments disclosed herein relate to systems, devices, and methods configured to facilitate reductions in the cost of performing encoding and decoding operations used in deoxyribonucleic acid (DNA) data storage systems. The embodiments also facilitate reducing errors in the encoding and decoding operations while accounting for a code structure used during those processes by constructing and using insertion-deletion-substitution (IDS) trellises for multiple traces.

In some embodiments, after a data message sequence, which includes multiple symbols (e.g., ones and zeros), has been encoded into a DNA sequence (e.g., combinations of A-C-G-T) and after the DNA sequence has been synthesized into multiple DNA strands, a DNA sequencing channel is used to randomly sample and sequence K (i.e.

a number greater than 1) DNA strands included among the multiple DNA strands. The sequencing process results in a generation of K noisy traces of the DNA sequence. Furthermore, the DNA sequencing channel is modeled as a simplistic zeroth order approximation in the form of a so-called "IDS channel." The embodiments also independently construct a corresponding trellis for each respective noisy trace in the K noisy traces. Consequently, K trellises are independently constructed. Here, the IDS channel is modeled as a finite state machine (FSM) to construct each of the K trellises. The embodiments then run a forward-backward algorithm on each of the K trellises to compute posterior marginal probabilities for vertices included in each of the K trellises. The embodiments also compute an estimate of the data message sequence by iterating through the following steps until all estimated symbols forming the estimate of the data message sequence are determined.

Specifically, for a given symbol that is to be included in the estimate of the data message sequence, the embodiments compute a current probability-based belief that each of the K trellises has regarding what the given symbol should be. Consequently, multiple current probability-based beliefs are computed. Here, the current probability-based beliefs are computed based on the posterior marginal probabilities included in each of the K trellises. The embodiments also select a particular symbol as the given symbol based on an aggregation of the current probability-based beliefs. The embodiments identify certain vertices in each of the K trellises. Notably, these certain vertices are identified as a result of the certain vertices disagreeing, based on those certain vertices' corresponding posterior marginal probabilities, with the decision to select the particular symbol as the given symbol. Those certain vertices are then updated by updating the posterior marginal probabilities for those certain vertices based on the decision. Forward passes are performed on the K trellises to update the K trellises based on updating the certain vertices. Additionally, the embodiments add the particular symbol to the estimate of the data message sequence.

This Summary is provided to introduce a selection of concepts in a simplified form that are further described below in the Detailed Description. This Summary is not intended to identify key features or essential features of the claimed subject matter, nor is it intended to be used as an aid in determining the scope of the claimed subject matter.

Additional features and advantages will be set forth in the description which follows, and in part will be obvious from the description, or may be learned by the practice of the teachings herein. Features and advantages of the invention may be realized and obtained by means of the instruments and combinations particularly pointed out in the appended claims. Features of the present invention are defined by the appended claims and will become more fully apparent from the following description or may be learned by the practice of the invention as set forth hereinafter.

BRIEF DESCRIPTION OF THE DRAWINGS

In order to describe the manner in which the above-recited and other advantages and features can be obtained, a more particular description of the subject matter briefly described above will be rendered by reference to specific embodiments which are illustrated in the appended drawings. Understanding that these drawings depict only typical embodiments and are not therefore to be considered to be limiting in scope, embodiments will be described and explained with additional specificity and detail through the use of the accompanying drawings in which:

Figure 1A illustrates an example process for storing binary data on DNA molecules.
Figure 1B illustrates how different errors can occur through the DNA data storage process.
Figure 1C provides another illustration of how different errors may occur.
Figure 1D provides yet another illustration of how different errors may occur.
Figure 2 illustrates an example of a probabilistic finite state machine (FSM).
Figure 3 illustrates an example trellis that is generated based on the FSM.
Figure 4 illustrates a technique for moving input and output points.
Figure 5 illustrates an example trellis that is generated for three input cycles.
Figure 6 illustrates two input cycles in an insertion, deletion, substitution (IDS) trellis for one trace.
Figure 7 illustrates a multi-trace trellis.
Figure 8 illustrates a graph based on real data showing a comparison in performance between multiple different error correcting techniques.
Figure 9 illustrates a graph based on simulated data showing comparisons in performance between multiple error correcting techniques.
Figure 10 illustrates another example of a trellis.
Figures 11A and 11B illustrate a flowchart of an example method for using trellis based reconstruction algorithms and inner codes for improving DNA data storage.
Figure 12 illustrates an example computer configured to perform any of the disclosed operations.

DETAILED DESCRIPTION

The following paragraphs will provide a sample overview of some of the high-level principles that are taught by this disclosure. Various features and technical improvements will also be described. Following this high-level overview, the disclosure will delve into specific details regarding how these benefits are achieved.

Generally, the disclosed embodiments are focused on improving the use of inner redundancy (i.e. inner codes) in order to reduce errors in DNA data storage models. To do so, the embodiments design decoding algorithms that take into account coding structure and the kinds of errors that may occur using that coding structure. Because of redundancy, the embodiments are able to resolve errors, thereby leading to a more accurate end result.

Some traditional systems deal with insertion-deletion-substitution (IDS) errors for only a single read (or "trace") per type of DNA sequence. To clarify, those traditional systems are inadequate when attempting to perform multiple reads and comparisons on a single type of DNA sequence. The embodiments are able to leverage situations in which redundancy within a DNA sequence is present (i.e. the use of inner codes) as well as scenarios where multiple copies of that DNA sequence are available (i.e. the use of outer codes). Using convolutional codes, the embodiments can provide or build an "optimal decoder" that optimizes (e.g., reduces) error rates given a particular code design. Using the disclosed principles, the embodiments are able to increase the probability of successfully and accurately recovering the binary data embedded in a DNA molecule.

As a point of clarification, although some trace reconstruction algorithms operate on multiple reads, the disclosed embodiments are different than those traditional systems. Specifically, one point of distinction is the following: traditional systems either (i) leverage redundancy that is present when operating on a single read or (ii) operate on multiple reads but without leveraging redundancy that is present in each strand. The disclosed embodiments, on the other hand, can operate on multiple reads while also exploiting the redundancy that is present in each strand. Additionally, it should be noted how multiple copies of a DNA sequence are available because of the underlying technology itself, not simply because of the redundancy in the outer codes. One contribution presented herein is focused on the inner code, whose encoding and decoding are independent of the outer code.

As will be discussed in more detail later, the embodiments do not strictly focus on maximizing the probability of recovering an entire binary data stream; rather, the embodiments successfully minimize the Hamming distance of the output relative to the original input. When an incorrect decoding does occur, those incorrections are very minimal and can be resolved by relying on outer codes. Additional details on these features will be provided later.

The embodiments also leverage the use of a "trellis." As a brief introduction, a trellis is a technique or visualization that enumerates all of the possible paths in a graphical model (e.g., a finite state machine). Enumerating all of the possible paths enables one to discern which possible events can occur as well as what possible errors can occur throughout the whole process, such as from the initial data segmentation process in which a file is broken down into discrete bits, through the synthesis and sequencing process, all the way to the final decoding process.

So, the trellis operates as a representation of the entire process from initial encoding to final decoding. In essence, the trellis can be used to outline all the possible errors (e.g., IDS errors) that might occur. As will be described in this disclosure, the recited trellis is different than traditional deterministic trellises because the recited trellises rely on statistical possibilities as opposed to deterministic outcomes. To clarify, IDS errors occur at random and are not determinate, thereby causing the recited finite state machines and corresponding trellises to rely on probabilities as opposed to certainties.

As recited earlier, multiple copies are typically generated for each DNA strand. The sequencing phase involves randomly sampling DNA strands and identifying the base sequences. Often, multiple copies of the same DNA strand will be sequenced. In accordance with the disclosed principles (and as will be described in more detail later), a separate trellis is built for each strand that is read (i.e. a "trace") during sequencing, resulting in a multi-trellis approach. The observations that each trellis generates will be different (e.g., because of IDS errors), so a different trellis is generated for each read, even for redundant reads. The information from those common trellises is then intelligently combined together to resolve errors.

That is, the multiple trellises can be merged or compared one to another to determine or estimate what the final output is (i.e. what the resulting estimated binary data is). The embodiments improve how the comparison and merging is performed to result in improved error correction. Notably, the embodiments also operate by avoiding decoding an entire sequence and then attempting to resolve or combine that sequence with other sequences at the end of the process. Instead, the embodiments perform the combining or merging in a progressive manner, as will be discussed later.

By way of a quick explanation, the embodiments estimate the bases in order from the beginning. As an example, suppose there are four reads of a particular sequence using four instances of a decoder. If three of the four decoders agree on a particular base (e.g., say the three decoders identified an "A" base), then it can be inferred that the base is likely correct (i.e. there is a high probability that "A" is the correct base in the sequence). This process can be performed progressively over the length of the sequence.

In contrast, if the process involved separately decoding the four sequences in their entirety and then trying to merge the resulting decoded sequences at the end, it may be the case that one of the decoders might have lost its sequence synchronization somewhere throughout the process. Consequently, that decoder's entire sequence may become worthless or questionable, resulting in increased read and coverage costs.

By causing the decoders to progressively decode together in a synchronized manner, the embodiments can keep the decoding processes all in alignment, thereby leading to an improved ability to resolve errors "in the moment" as opposed to "at the end." In this regard, this disclosure describes a technique for enabling multiple decoders to collaborate on how to decode common DNA sequences via a synchronization of input and output pointers. All of these features will be discussed in detail later.

DNA Data Storage Model

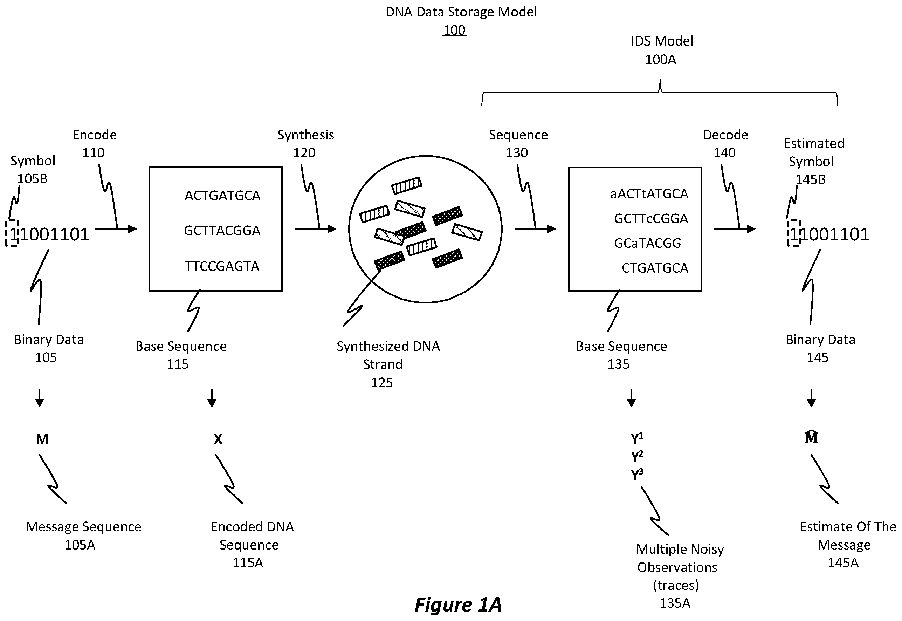

Attention will now be directed to Figure 1A, which illustrates an example of a DNA data storage model 100 for storing binary data on DNA molecules and for recovering that binary data from the DNA molecules. Initially, a data file is segmented into its constituent binary parts, resulting in the binary data 105. The binary data 105 is then subject to an encoding process (e.g., encode 110) to generate a desired base sequence 115 comprising various combinations of the A-C-G-T nucleotide bases. Typically, the length of each DNA sequence is relatively short, such as around 150 bases or nucleotides per sequence; the range is usually between 100-300 bases per sequence. The encoding process involves mapping the ones and zeros of the binary data 105 into the A-C-G-T nucleotides of DNA. As a rudimentary example, the binary data 105 can be split into two-bit units of data (e.g., 00, 01, 10, 11) to map to the four nucleotide bases. As an example, "00" can map to "A;" "01" can map to "C;" "10" can map to "G;" and "11" can map to "T." Of course, other mapping / encoding techniques may be used.

With the binary data 105 now mapped to generate sequences of nucleotides, the DNA data storage model 100 shows how actual DNA strands can be generated via a synthesis 120 process to create the actual synthesized DNA strands (e.g., synthesized DNA strand 125). Thousands or even potentially millions of copies of each DNA strand may be generated, resulting in duplicate copies (each of which may be prone to error). With the DNA strands now synthesized, these strands can be placed in storage for a prolonged period of time.

When the time comes for reading the data stored on the DNA strands, the DNA data storage model 100 shows a sequencing process (e.g., sequence 130). At a high level, the sequence 130 process is the opposite process of the synthesis 120 process. There are numerous different technologies available to perform sequencing. One example is the Illumina sequencing process. Another example is the Nanopore process. The Nanopore process typically has higher error rates in reading (aka sequencing) the DNA strands. To illustrate, the Illumina sequencing process often achieves about a 1% error rate while the error rate in the Nanopore sequencing process can be as high as 10%. Nanopore sequencing is still quite valuable, however, because of its simplicity, lower cost, and small size.

Generally, the sequencing process includes sampling the DNA strands (i.e. oligonucleotides) in a random manner. This random sampling process may lead to scenarios where a particular DNA strand is never sampled (i.e. a so-called "lost sequence") and/or it may lead to scenarios where multiple ones of the same DNA strand are sampled.

Because the DNA strands are sampled in a random manner, there is no initial ordering that occurs with the sequencing process. To compensate for this lack of ordering, an index can be encoded with a base sequence to indicate the order or positioning of a particular base sequence relative to other base sequences.

As a result of performing the sequence 130 operation, the base sequence 135 is now known. During the decoding phase (i.e. decode 140), the nucleotides in the base sequence 135 are mapped back into a binary format to form the binary data 145. As will be described later, the DNA sequencing channel can be modeled as a simplistic zeroth order approximation in the form of an IDS channel 100A.

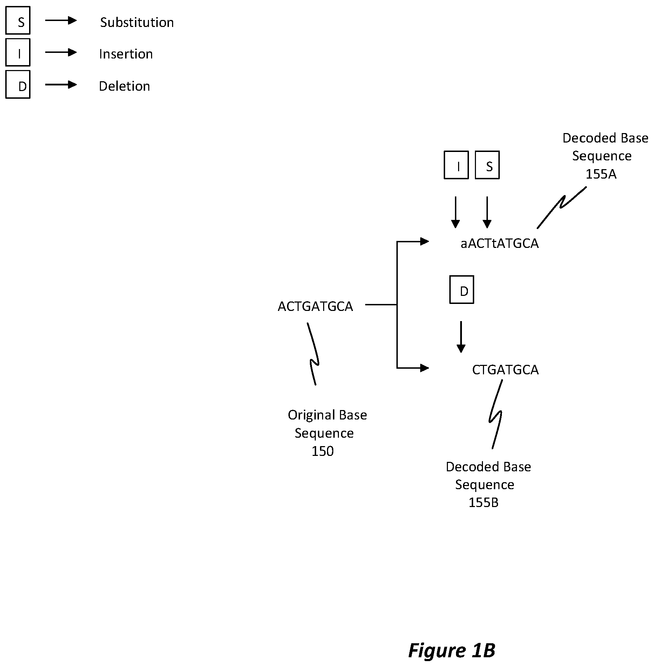

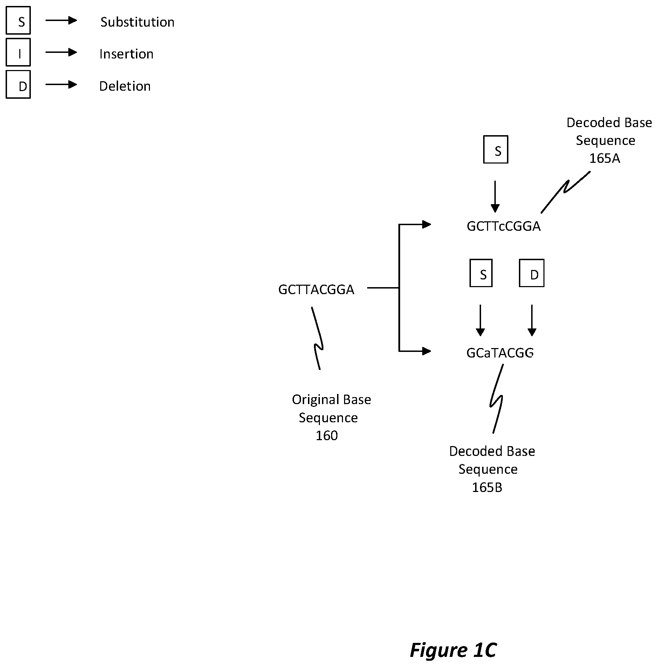

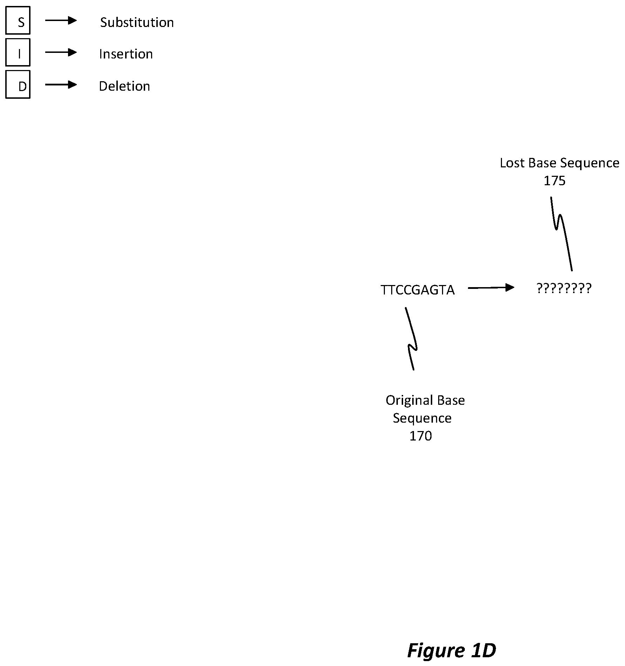

Each phase or process involved in the DNA data storage model 100 can lead to errors. For example, errors may occur during the encoding, synthesis, storage, sequencing, and even decoding phases. The phases most prone to IDS errors are the synthesis, storage, and sequencing phases. Such errors occur based on the types of technologies used, as described earlier with respect to Nanopore and Illumina techniques. Figures 1B, 1C, and 1D illustrate examples of such IDS errors. In order to successfully recover from the data, the disclosed embodiments provide an improved error recovery technique that is reliant on coverage (e.g., how many DNA strand samples are needed to decode a base sequence accurately) and redundancy (e.g., redundancy of bases within a single DNA strand). These error recovery techniques are provided by the inner and outer codes.

Figure 1B shows an original base sequence 150, which is representative of one base sequence in the base sequence 115 of Figure 1A. This original base sequence 150 includes the following combination of nucleotides: ACTGATGCA. As a result of performing the synthesis 120 and sequence 130, one will notice how there are two resulting base sequences that correspond to the original base sequence 150, as shown in the base sequence 135. Specifically, those two base sequences include decoded base sequence 155A (i.e. aACTtATGCA) and decoded base sequence 155B (i.e. CTGATGCA). Use of the lower case letters in these sequences represents an error.

The I, D, and S letters enveloped by the rectangles reflect errors that have occurred. For example, the decoded base sequence 155A includes an insertion (I) error (the extra "a" at the beginning of the sequence is an insertion) and a substitution (S) error (e.g., the "t" improperly replaced the "G" in the original sequence). Similarly, the decoded base sequence 155B includes a deletion (D) error (e.g., "A" is missing at the beginning of the sequence).

Figure 1C shows an original base sequence 160, which corresponds to the GCTTACGGA sequence in Figure 1A. Here, two resulting DNA strands are sequenced, as shown by decoded base sequence 165A and decoded base sequence 165B. For the decoded base sequence 165A, a substitution (S) error has occurred, where a "c" has replaced a "A." Similarly, for the decoded base sequence 165B, a substitution (S) error has occurred (e.g., "a" replaced the "T") and a deletion (D) error has occurred (e.g., the sequence is missing the "A" at the end).

Figure 1D shows an original base sequence 170. In this example, however, as a result of performing the random sampling during the sequencing phase, no DNA strands for this sequence were identified, resulting in a lost base sequence 175.

Accordingly, Figures 1A, 1B, 1C, and 1D illustrate the inner and outer code architecture for DNA binary data storage. During the process of information retrieval, the encoded DNA strands are read or "sequenced" using a sequencing technology, such as Illumina or Nanopore sequencers. This sequencing operation outputs many noisy copies of base sequences. Given this architecture, it is desirable to design an encoder (e.g., an encoder that performs the encode 110 operation) and a corresponding decoder (e.g., a decoder that performs the decode 140 operation) with the knowledge of which sequencing technology is being used. Using the inner and outer codes helps prevent (or at least provide an option for recovering from) the scenarios presented in Figures 1B, 1C, and 1D.

With regard to the mathematical equations presented herein, Figure 1A also illustrates some reference labels. Specifically, the binary data 105 is indicated with an **"M"** and is a "message sequence" 105A that includes multiple symbols, as shown by the "1" symbol 105B. One of the base sequences in base sequence 115 (e.g., perhaps the base sequence "ACTGATGCA") is indicated with a **"X"** and is an "encoded DNA sequence" 115A. Any base sequences in base sequence 135 corresponding to **"X"** is indicated with the series "$Y^1Y^2Y^3$" and refers to "multiple noisy observations" 135A (aka "noisy traces"). For instance, **"X"** might be the base sequence "ACTGATGCA" and the "multiple noisy observations" 135A for **"X"** would include $Y^1$ = "aACTtATGCA" and $Y^2$ = "CTGATGCA." The resulting binary data 145 is indicated with a "$\hat{M}$" and is an "estimate of the message" 145A and includes a number of estimated symbols, such as estimated symbol 145B.

DNA Sequencing Channel

As described earlier, the exact error profile of the noisy observations or errors is dependent on the DNA sequencing technology used (e.g., Illumina, Nanopore, etc.). However, exactly modeling this error profile is tedious and often impracticable. Moreover, DNA sequencing technologies are evolving at a rapid pace, and exactly modeling the error profiles does not give a future-proof approach to the problem. Instead, a common practice is to consider a simplistic zeroth order approximation for the sequencing channel, by modeling it as an Insertion-Deletion-Substitution (IDS) channel, which will be defined shortly. Generally, the IDS channel is the channel that builds the "multiple noisy observations" 135A (aka noisy traces) in Figure 1A. Or, in other words, the IDS channel builds each one of the $Y^1Y^2Y^3$ shown in Figure 1A. Notably, the disclosed principles also naturally fit in more complex approximations for the "channel model" (this term is used to refer to the process of modeling of the sequencing channel). For instance, insertions and deletions often occur in "bursts," and such events can be captured by a first-order Markov model. Beneficially, the disclosed decoder can easily be modified to accommodate for this model.

Problem Set-Up - IDS Channel

The Insertion-Deletion-Substitution (IDS) channel is defined by four non-negative parameters $p_{ins}$, $p_{del}$, $p_{sub}$, $p_{rep}$ with $p_{ins}+p_{del}+p_{sub}+p_{rep}$ = 1. Given an $N$-length input sequence $X = X_1X_2...X_N$ from an alphabet $\Sigma$ (e.g., the nucleotide bases A-C-G-T), the IDS channel sequentially takes in one input symbol (e.g., a nucleotide base) at a time and performs one of the following operations in order to build a sequence (e.g., the "multiple noisy observations" 135A from Figure 1A):

Insertion: with probability $p_{ins}$, inserts a symbol from $\Sigma$ uniformly at random and stays at the same input symbol.
Deletion: with probability $p_{del}$, deletes the input symbol and goes to the next input symbol.
Substitution: with probability $p_{sub}$, substitutes the input symbol with a different symbol from $\Sigma$ uniformly at random and goes to the next input symbol.
Replication: with probability $p_{rep}$, does nothing to the input symbol and goes to the next input symbol.

Once the IDS channel exhausts the input symbols, the IDS channel then outputs the modified sequence. The output of the IDS channel is referred to herein as a "trace." A notation and definition guide is provided below.

| IDS channel | Insertion-Deletion-Substitution channel |
|---|---|
| Trace | Output of the IDS channel |
| Upper-case letters (e.g., $X$) | A random variable/integer constant |
| Lower-case letters (e.g., $x$) | A variable |
| Bold-face symbols (e.g., $\mathbf{x}$) | A sequence/vector |
| Bold upper-case symbols (e.g., $\mathbf{X}$) | A random vector |
| Superscripts (e.g., $\mathbf{Y}^k$) | $k^{th}$ trace |
| Subscripts (e.g., $x_n$) | $n^{th}$ symbol of sequence $\mathbf{x}$ |
| TR | Trace reconstruction |
| MAP | Maximum a-posteriori |
| Improved BMALA | Improved bitwise majority alignment with lookahead |
| CC | Convolutional code |
| FSM | Finite-state machine |
| Trellis BMA | Trellis bitwise majority alignment |

Notation And Definition Guide

Problem Set-Up - Trace Reconstruction (TR)

One overall goal of trace reconstruction is to compute an estimate $\hat{X}(\mathbf{Y}^1, \mathbf{Y}^2, ..., \mathbf{Y}^K)$ (e.g., "estimate of message" 145A in Figure 1A) of the input sequence $\mathbf{X}$ (e.g., message sequence 105A in Figure 1A) from multiple independent traces $\mathbf{Y}^1, \mathbf{Y}^2...,\mathbf{Y}^K$ of $\mathbf{X}$ (e.g., "multiple noisy observations" 135A in Figure 1A). The precise goal varies depending on the application. For instance, one might aim for exact sequence reconstruction, i.e., minimize $\mathbf{Pr}(\hat{X} \neq \mathbf{X})$ (e.g., minimize the probability that the "estimate of the message" 145A in Figure 1A is equal to the "message sequence" 105A). In accordance with the disclosed principles, however, it is desirable to minimize the number of symbol mismatches, i.e.,

$$min \sum_{n=1}^{N} \Pr(\widehat{X}_n \neq X_n) \qquad (\text{P.1})$$

The quantity $\sum_{n=1}^{N} \Pr(\widehat{X}_n \neq X_n)$ is called the expected "Hamming distance" between the actual and estimated sequence. The reason for solving (P.1) is that typically outer codes are used in DNA storage systems to account for missing sequences, and the same codes are capable of correcting substitution type errors. In essence, by solving (P.1) it is possible to convert "deletion" and "insertion" errors into "substitution" type errors, for which codes and decoding algorithms are well studied.

An optimal solution to (P.1) is to pick the most likely value for each symbol in $\mathbf{X}$ given the observations. Such an estimate is called a "symbolwise maximum-aposteriori" (MAP) estimate, and the disclosed principles are able to provide an exact algorithm to accomplish this task.

Problem Set-Up - Coded Trace Reconstruction (TR)

Consider a code which maps a message sequence $\mathbf{M} =M_1M_2...M_L$ (e.g., message sequence 105A in Figure 1A) to a codeword $\mathbf{X} = X_1X_2...X_N$ (e.g., encoded DNA sequence 115A in Figure 1A). One goal of coded TR is to compute an estimate $\hat{M}(\mathbf{Y}^1, \mathbf{Y}^2, ..., \mathbf{Y}^K)$ (e.g., estimate of the message 145A in Figure 1A) of the message sequence $\mathbf{M}$ from multiple independent traces $\mathbf{Y}^1, \mathbf{Y}^2, ..., \mathbf{Y}^K$ of $\mathbf{X}$ (e.g., multiple noisy observations 135A in Figure 1A). This formulation fits in naturally with the DNA storage architecture described with respect to Figure 1A. As with TR, one goal of the disclosed embodiments is to minimize the expected Hamming distance, i.e.,

$$min \sum_{l=1}^{L} \Pr(\widehat{M}_l \neq M_l) \qquad (\text{P.2})$$

As with TR, one optimal solution is to pick the most likely value for each symbol in $\mathbf{M}$ given the traces.

Some systems use an identity map in place of an inner code (notably, outer codes are still used even if inner codes are otherwise not usable) to encode a message sequence (i.e. a binary file) to a DNA sequence. For the decoder, such systems use a TR algorithm called Improved BMALA (i.e. improved bitwise majority algorithm with lookahead) to estimate the DNA sequence and the subsequent, resulting, or decoded message sequence.

In particular, improved BMALA attempts to estimate each symbol of **X** sequentially. It uses a pointer for each of the traces, and uses the pointed symbols to form a plurality vote to determine the next symbol of **X.** For the traces that do not agree with the plurality vote, improved BMALA attempts to decide the reason for disagreement (e.g., was there an insertion, deletion, or substitution) by looking ahead a few symbols and then moves the corresponding pointers accordingly. If the algorithm cannot decide on any reason for disagreement, it discards the trace temporarily and attempts to bring that trace back at a later point in time.

Improvements And Benefits

Although improved BMALA is seen to perform well as a standalone TR algorithm, it is not the optimal solution for solving (P.1). Moreover, improved BMALA does not take into account the structure of the code used, if any. Ideally, it is desirable to use an algorithm that solves the coded TR problem (P.2) exactly. Beneficially, such an algorithm also solves (P.1) exactly.

Advantageously, the disclosed algorithms are able to exactly solve (P.2) in situations where the encoder can be modeled as a (possibly time-varying) deterministic finite-state machine. Such an encoder model encompasses a variety of useful codes, such as repetition codes, convolutional codes (CC), and watermark codes.

The disclosed embodiments also beneficially provide a heuristic (referred to herein as "Trellis BMA") that marries i) the idea from Improved BMALA and ii) the core idea of the exact algorithm for solving (P.2). This heuristic is much faster than the exact algorithm used to solve (P.2) and has significant performance improvements over improved BMALA, even when used as a standalone TR algorithm (with an identity map as the inner code).

As a general description, the disclosed embodiments are able to identify that the "code + channel system" can be captured by a probabilistic finite-state machine (FSM), which equivalently defines a hidden Markov model (HMM) and a directed acyclic graph called a "trellis."

Given this formulation, the optimal solution to (P.2) reduces to computing the marginal distribution of the hidden states in the HMM. This is accomplished by a dynamic programming approach called the "forward-backward algorithm" (also called BCJR algorithm) that is performed on the trellis. Beneficially, the complexity of the forward-backward algorithm on the trellis is linear in the number of edges in the trellis, thereby significantly reducing the amount of computations. While it is true that the complexity is linear in the number of edges in the trellis, that linear relationship may result in the number of edges in the trellis potentially growing unmanageably large with the number of traces. To mitigate such occurrences, the disclosed trellis algorithms circumvent this issue by constructing smaller trellises (one from each trace) and then making these small trellises communicate with each other to improve their final estimate.

As another benefit, the disclosed model simultaneously accounts for multiple traces. Moreover, for multiple traces, the disclosed trellis construction has exponentially fewer edges compared to other types of trellises that are used in a "force-fit" manner in an inadequate attempt to extend existing ideas to multiple traces.

Additionally, the disclosed principles focus on a solution to the TR problem through the use of a probabilistic inference point-of-view. The Trellis BMA algorithm epitomizes this probabilistic approach to TR and coded TR by generalizing and by lending a probabilistic perspective to an existing TR algorithm, thereby improving its performance as well as flexibility of usage.

Probabilistic FSM and Trellis

As introduced above, the disclosed principles rely on the use of a trellis structure. Further details on this structure will be provided momentarily, but it should first be noted how the disclosed description of a trellis varies from the usual descriptions in coding theory. This variation beneficially admits a larger class of input-output distributions, such as the one that describes the IDS channel. As such, the disclosed trellis structure is unique and provides substantial benefits to the technical field. The disclosed trellis is also not based on deterministic data; rather, it is based on probabilities.

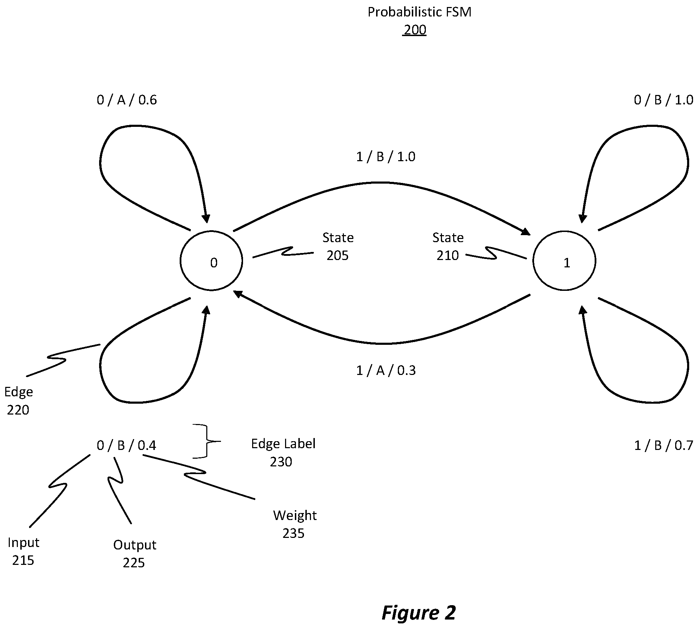

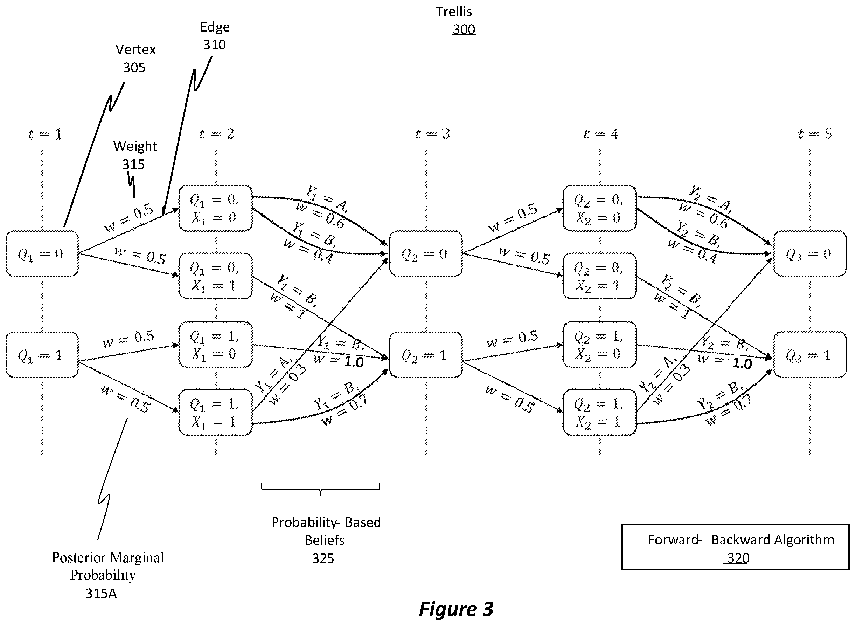

A probabilistic FSM is a FSM that accepts an input symbol and outputs a symbol while making a state transition. The output and next state are probabilistic functions of the input symbol and current state. A trellis is a directed acyclic graph (DAG) that tracks the evolution of the FSM states over time. Figure 2 shows an example of a probabilistic FSM 200. The trellis corresponding to the probabilistic FSM 200 expands and repeats the state-space over time and is illustrated in Figure 3. Notably, the trellis 300 of Figure 3 can itself be envisioned as a larger probabilistic FSM, where the state-space includes a time or "stage" component.

Returning to Figure 2, Figure 2 illustrates an example of a probabilistic FSM 200. This probabilistic FSM 200 includes two states, namely state 205 (i.e. a state of "0") and state 210 (i.e. a state of "1").

In this scenario, the probabilistic FSM 200 is configured to accept an input 215, which is formed of a symbol $x \in \{0, 1\}$. Notably, the input symbols can be generated uniformly at random in this example. The probabilistic FSM 200 also includes a number of edges, such as edge 220, which connects one state either to itself or to another state. For example, the edge 220 is connecting state 205 to itself. More generally, an edge (e.g., edge 220) connects state $s$ to state $s'$ and also has an associated input 215 (e.g., input symbol $x$) and an output 225 (e.g., an output symbol $y \in \{A,B\}$). The edge label 230 corresponds to $x / y / \Pr(y, s' \mid s, x)$, and the weight 235 (i.e. the likelihood that a certain output will result based on the state and the input) of each edge is defined to be $\mathbf{Pr}(y, s' \mid x, s)$.

As an example, suppose the current state of the probabilistic FSM 200 is at state 205 and further suppose an input of 0 is received. In this example scenario, there is a 60% likelihood or "weight" that the output will be "A." On the other hand, there is a 40% likelihood that the output will be "B." In this scenario, there is a 100% likelihood that the next state will be the "0" state.

As another example, suppose the current state of the probabilistic FSM 200 is at state 210 and further suppose an input of 1 is received. In this example scenario, there is a 70% likelihood that the output will be "B" and the next state will also be state 210. On the other hand, there is a 30% likelihood that the output will be "A" and the next state will be state 205. Accordingly, Figure 2 illustrates an example of a probabilistic FSM.

As indicated earlier, a trellis is a directed acyclic multigraph (i.e. a graph with multiple edges between the same pair of vertices). A trellis describes the joint distribution of an observable set of variables $\mathbf{Y} = (Y_1, Y_2, ..., Y_M)$ and a latent or hidden sequence of states $\mathbf{S} = (S_1, S_2, ..., S_N)$. Each state $S_i$ defines the stage $i$ of the trellis. The support of each $Y_j$ is a finite set $\mathbf{y}_j$. Likewise, the support of $S_i$ is a finite set $\mathbf{S}_i$. The details of a trellis will now be described with respect to Figure 3.

Figure 3 shows a resulting trellis 300 for the probabilistic FSM 200 of Figure 2, where the trellis 300 models all possible events with 2 inputs to the probabilistic FSM 200. For example, at $t = 1$, the state of the trellis 300 is the state $Q_1$ of the probabilistic FSM 200 before the probabilistic FSM 200 accepts a first input symbol $X_1$. Once the probabilistic FSM 200 accepts the first input symbol, the trellis 300 pushes the input symbol to an input buffer (e.g., modeled as a part of the state-space at $t = 2$). The edge weights from $t = 1$ to $t = 2$ model the input distribution.

The probabilistic FSM 200 then probabilistically transits to the next state $Q_2$ while emitting a first output symbol $Y_1$. The edges connecting stages $t = 2$ and $t = 3$ capture that result. The edge labels $Y_1 = y$ indicate the output symbol emitted while the edge weight w is equal to $\Pr(Y_1 = y, Q_2 \mid X_1, Q_1)$. This cycle is followed once more until $Y_2$ is emitted and the probabilistic FSM 200 transitions to its end state $Q_3$.

It should be noted that the vertices (i.e. the rounded edge rectangles shown in Figure 3, such as vertex 305) of the trellis 300 correspond to all possible realizations of each individual state. In other words, each vertex is labeled $S_i = s$, where $s \in \mathbf{S}_i$. Throughout this disclosure, a vertex is identified using its label $S_i = s$.

An edge (i.e. the arrowed lines in Figure 3, such as edge 310) in the trellis 300 connects a set of vertices, thereby transitioning the trellis 300 from one labeled state $S_i = s$ to a different labeled state $S_i = s'$, where $i < N$ (termed intra-stage edges), or they connect vertices with labels $S_i = s$ and $S_{i+1} = s'$, where $i < N$ (termed inter-stage edges). Note that the last stage corresponding to $S_N$ has no outgoing edges. The trellis 300 is also configured to disallow intra-stage edges at the first stage (i.e. $Q_1$). To do so, the trellis 300 can be prepended with a dummy stage with exactly one vertex, and vertices can be connected to the first stage of the trellis.

An edge can either have no label (unlabeled edge) or can be labeled $Y_j = y$ where $y \in \mathbf{y}_j$. Multiple edges can have the same label. However, two edges connecting the same pair of vertices cannot have the same label. In such cases, one of the two identical edges is simply removed.

Every edge in the trellis 300 is weighted, as shown by weight 315 (aka a posterior marginal probability 315A) on the edge 310. The weight of an unlabeled edge connecting vertices with labels $v$ and $v'$ is equal to $\Pr(v' \mid v)$. By way of example, an edge connecting $S_i = s$ to $S_{i+1} = s'$ has a weight $\Pr(S_{i+1} = s' \mid S_i = s)$. The weight of an edge with label $l$ connecting vertices with labels v and $v'$ is equal to $\Pr(l, v' \mid v)$. As another example, an edge with label $Y_j = y$ connecting $S_i = s$ to $S_{i+1} = s'$ has a weight $\Pr(Y_j = y, S_{i+1} = s' \mid S_i = s)$. As will be discussed in more detail later, a forward-backward algorithm 320 can be run on the trellis 300 in order to compute posterior marginal probabilities (i.e. the weights) for each of the vertices.

For a vertex $S_i = s$ with $i < N$, the weights of all outgoing edges should sum to 1. For example, the vertex 305 has two edges extending away from it, as shown by edge 310 and another edge (not labeled in Figure 3). The sum of the weights for these two edges equals 1.

In a trellis (e.g., trellis 300), no path contains two edges corresponding to two realizations of the same "observable." In other words, no path contains a pair of edges with labels $Y_j = s$ and $Y_j = s'$, for any $j \in \{1, 2, ..., M\}$ and s, $s' \in \mathbf{y}_j$. The weight $w(p)$ of a path $p$ is defined to be the product of weights of constituent edges. Notably, a trellis (e.g., trellis 300) is also defined by a fixed prior distribution on its initial state $S_1$.

The above description of a trellis induces a joint distribution on **(S, Y).** Such a model is also called a Hidden Markov Model (HMM), because given the current state, the observable that follows is independent of the history. Each path $p$ connecting a vertex $S_1 = s_1$ to vertex $S_N = s_N$ in the trellis corresponds to a particular realization of states $(S_1 = s_1, ..., S_N =$

$s_N$) and observables ($Y_1 = y_1, ..., Y_M = y_M$). Moreover, path weight $w(p)$ of a path $p$ traversing the sequence of states ($S_i = s_i, ..., S_i' = s_i'$) and through edges with labels ($Y_j = y_j, j \in \mathcal{J}$), $\mathcal{J} \subseteq \{1, 2, ..., M\}$ is defined in the following manner:

$$w(p) = \Pr(S_{i+1} = s_{i+1}, ..., S_i' = s_i', (Y_j = y_j, j \in \mathcal{J}) \mid S_i = s_i) \qquad \text{Eq. (1)}$$

Now, consider a path $p$ connecting a vertex of stage 1 $S_1 = s_1$ to a vertex of the last stage $S_N = s_N$ passing through the edges with labels $Y_1 = y_1, ... , Y_M = y_M$. From (1), the following is obtained:

$$\Pr(S_1 = s_1)w(p) = \Pr(S_1 = s_1, ..., S_N = s_N, Y_1 = y_1, ..., Y_M = y_M)$$

Consequently, each path connecting stage 1 and stage $N$ calculates an associated $\Pr(\mathbf{S} = \mathbf{s}, \mathbf{Y} = \mathbf{y})$.

It should be noted that it is possible that a path connecting stages 1 and N need not contain any edge with a label corresponding to some observable $Y_j$. Indeed, such cases are accounted by appending a dummy "empty" character ø to $\boldsymbol{y_j}$ and assuming that $Y_j = \emptyset$ in such a path.

Marginalization Via The Forward-Backward Algorithm

Given a particular realization y = ($y_1, ..., y_M$) for the set of observables **Y**, one question to ask is: what is the posterior distribution of a given state $S_i$? This section is devoted to answering that question. In other words, this section describes an algorithm called the "forward-backward algorithm" (also called BCJR algorithm) to compute $\Pr(S_i = s \mid \mathbf{Y} = \mathbf{y})$, $\forall\, i, s$ in $O(E)$ where $E$ is the number of edges in the trellis.

The high-level idea, given the description of the trellis, is as follows. For each $i, s$, it is possible to compute $\Pr(S_i = s, \mathbf{Y} = \mathbf{y})$ and normalize across all realizations of $S_i$ to obtain $\Pr(Si = s \mid \mathbf{Y} = \mathbf{y})$ for each $s$, as illustrated below in (2).

$$\Pr(\mathbf{S_i} = \mathbf{s}, \mathbf{Y} = \mathbf{y}) = \sum_{s_1,...,s_{i-1},s_{i+1},...,s_N} \Pr(\mathbf{S_1} = \mathbf{s_1}, ..., \mathbf{S_i} = \mathbf{s}, ..., \mathbf{S_N} = \mathbf{s_N}, \mathbf{Y} = \mathbf{y})$$

$$= \sum_{s_1} \left( \Pr(\mathbf{S_1} = \mathbf{s_1}) \sum_{s_2,...,s_{i-1},s_{i+1},...,s_N} \Pr(\mathbf{S_2} = \mathbf{s_2}, ..., \mathbf{S_i} = \mathbf{s}, ..., \mathbf{S_N} = \mathbf{s_N}, \mathbf{Y} = \mathbf{y} \mid \mathbf{S_1} \right.$$

$$\left. = \mathbf{s_1}) \right)$$

$$\text{Equation (2)}$$

The expression inside the parenthesis on the right-hand side of (2) is then interpreted in terms of summation of path weights in a modified trellis. Calculating this summation of path weights is accomplished by a pair of dynamic programs. The disclosure will now delineate the algorithm in four steps.

Step1: Modifying the trellis to explain the observations.

First, it is possible to modify the trellis so that the trellis describes the joint distribution **(S, Y = y).** This is done by retaining only the edges where the labels are compatible with the observations. In other words, it is possible to remove an edge with label $Y_j = y$ from the trellis if $\boldsymbol{y} \neq \boldsymbol{y_j}$. For a path $p$ connecting stages 1 and $N$ traversing the sequence of states ($S_1 = s_1, ..., S_N = s_N$), equation (3) below is the result:

$$\Pr(S_1 = s_1)w(p) = \Pr(S_2 = s_2, ..., S_N = s_N, \mathbf{Y} = \mathbf{y} \mid S_1 = s_1) \qquad \text{Eq. (3)}$$

Now, equation (2) is looped back to, which can be rewritten in the following manner:

$$\mathrm{Pr}(S_i = s, \boldsymbol{Y} = \boldsymbol{y}) = \left( \sum_{s_1, s_2, \dots, s_{i-1}} \mathrm{Pr}(S_1 = s_1) \, \mathrm{Pr}(S_2 = s_2, \dots, S_i = s, \boldsymbol{Y}_1 = \boldsymbol{y}_1 \mid S_1 = s_1) \right)$$

$$\left( \sum_{s_{i+1}, \dots, s_N} \mathrm{Pr}(S_i = s, \dots, S_N = s_N, \boldsymbol{Y}_2 = \boldsymbol{y}_2 \mid S_i = s) \right)$$

Equation (4)

where the observation $\boldsymbol{y}$ is split into two parts. In particular, the first part $\boldsymbol{y}_1$ collects the observations until state $S_i = s$, and the other part $\boldsymbol{y}_2$ consists of observations post $S_i = s$.

The term inside the first parenthesis on the right-hand side of (4) is interpreted as follows. Specifically, this term is equal to:

$$\sum_p \mathbf{Pr}(\boldsymbol{v}_{start}(\boldsymbol{p})) \boldsymbol{w}(\boldsymbol{p})$$

where the summation is over all paths $p$ that originate at stage 1 and end at $S_i = s$, and where $v_{start}(p)$ is the label of the origin of the path. Similarly, the term inside the second parenthesis on the right-hand side of (4) is interpreted as follows. Specifically, this is equal to:

$$\sum_p w(p)$$

where the summation is over all paths $p$ that originate at $S_i = s$ and end at stage $N$.

Step2: Computing the forward values for each state.

The dynamic program that computes $\Sigma_p \, \mathbf{Pr}(\boldsymbol{v}_{start}(\boldsymbol{p})) \boldsymbol{w}(\boldsymbol{p})$ will now be presented. Notably, the summation is over all paths $p$ that originate at stage 1 and end at vertex v, and $v_{start}(p)$ is the origin vertex of the path. This term is called the "forward value" of vertex v. In other words, equation (5) is developed:

$$F(\boldsymbol{v}) \triangleq \Sigma_p \mathbf{Pr}\big(\boldsymbol{v}_{start}(\boldsymbol{p})\big) \boldsymbol{w}(\boldsymbol{p}) \qquad\qquad (5)$$

where the summation is over all paths $p$ that originate at stage 1 and end at v, and where $v_{start}(p)$ is the label of origin vertex of the path.

Suppose a path $p$ traverses the alternating sequence of vertices and edges $(v_{start}(p), e_1, ..., v', e', v)$. Let $p'$ be the subpath of $p$ that terminates at $v'$. Thus $w(p) = w(p')w(e')$ and $w(e')$ is the weight of the incoming edge $e'$. One could equivalently sum over all in-edges $e'$ of v and then over all $p'$ that terminate at the head $v'$ of $e'$. In other words, equation (6) results:

$$F(v) = \sum_{p} \Pr\big(v_{start}(p)\big) w(p)$$

$$= \sum_{e'} \sum_{p'} \Pr\big(v_{start}(p')\big) w(p')w(e')$$

$$= \sum_{e'} F(v')w(e')$$

Equation (6)

where the summation is over all in-edges $e'$ of $v$ and where v'is the head of $e'$. Thus, the forward value of a vertex is described by a simple sum-product update rule, namely, it is the sum (over all in-edges) of the product of the in-edge weight and forward value of the corresponding in-neighbor.

To compute the forward values of all vertices, it is possible to first compute a topological ordering for the vertices of the trellis. Recall that the trellis is a DAG, so such an ordering always exists. Next it is possible to initialize the forward values of the vertices in stage 1; $F(S_1 = s) = \Pr(S_1 = s) \; \forall \; s \in \mathcal{S}_i$.

Next, it is possible to traverse the vertices in order and use the aforementioned sum-product update rule in (6) to compute $F(v)$ for all vertices in the trellis. Since each edge in the trellis is traversed exactly once when computing the forward values, the complexity of computing the forward values is $O(E)$, where $E$ is the number of edges in the trellis.

Note, the complexity of finding a topological ordering is $O(E)$ as well. As a consequence, finding this ordering will not affect the overall complexity.

Step3: Computing the backward values for each state.

The dynamic program that computes the second term in (4) will now be presented, where the second term is equal to $\Sigma_p w(p)$, where the summation is over all paths $p$ that originate at $v$ and end at stage $N$. This summation is called the backward value $B(v)$ of vertex $v$.

Analogous to the argument made for the forward values, it is possible to first consider a path $p$ that traverses the alternating sequence of vertices and edges $(v, e, v', ...)$. Let $p'$ be the subpath of $p$ that originates at $v'$. Thus w(p) = $w(p')w(e')$ and $w(e')$ is the weight of the outgoing edge $e'$. It is possible to equivalently sum over all out-edges $e'$ of $v$ and then over all $p'$ that originate at the tail $v'$ of $e'$. In other words, equation (7) is the result:

$$B(v) = \sum_{p} w(p)$$

$$= \sum_{e'} \sum_{p'} w(p')w(e')$$

$$= \sum_{e'} B(v')w(e')$$

Equation (7)

To compute the backward values of all vertices, the embodiments use the reverse topological ordering for the vertices of the trellis. Next, the embodiments initialize the backward values of the vertices in stage $N$. For each $s$, it is beneficial to ask the question "is $S_N = s$ reachable given the observations?"

If the answer is yes, the embodiments fix $B(S_N = s) = 1$, otherwise $B(S_N = s) = 0$.

Next, the embodiments traverse the vertices in the reverse topological order and use the aforementioned sum-product update rule in (7) to compute $B(v)$ for all vertices in the trellis. The complexity of computing the backward values is also $O(E)$, since each edge is traversed exactly once.

Step 4: Computing the posterior marginals using the forward and backward values.

Having computed the forward and backward values, it is now possible to rewrite (4) as

$$\Pr(S_i = s, \mathbf{Y} = \mathbf{y}) = F(S_i = s)B(S_i = s)$$

The above expression can be normalized to obtain the posterior marginal probabilities in the following manner:

$$\mathbf{Pr}(S_i = s | Y = y) = \frac{F(S_i = s)B(S_i = s)}{\sum_{s'} F(S_i = s')B(S_i = s')}$$

Complexity analysis.

Steps 1-3 above each traverse the trellis edges exactly once. Moreover, finding a traversal order for the trellis vertices is $O(E)$.
Step 4 iterates through the vertices so it is still $O(E)$. The number of vertices can be assumed to be at most the number of edges, without loss of generality; otherwise, the isolated vertices are removed since those states can never be reached. The time complexity of the forward-backward algorithm is therefore $O(E)$.

Coded TR using the multi-trace IDS trellis

Consider a message sequence $\mathbf{M} = M_1 M_2 \dots M_L$ (e.g., message sequence 105A from Figure 1A) which is mapped onto a codeword $\mathbf{X} = X_1 X_2 \dots X_N$ (e.g., encoded DNA sequence 115A in Figure 1A) using a (time-varying) deterministic FSM. Suppose K independent traces $\mathbf{Y}^1$, $\mathbf{Y}^2$, ..., $\mathbf{Y}^K$ of $\mathbf{X}$ are observed (e.g., "multiple noisy observations" 135A in Figure 1A). In this section, the disclosure will describe the computation of the symbolwise posterior probabilities:

$$\mathbf{Pr}(M_l = m | Y^1, Y^2, \dots, Y^K) \; \forall \, l, m$$

It is possible to then use this understanding to pick the most likely value for each symbol $M_l$. Such an estimator is then the optimal solution for (P.2).
Given the definition of the trellis DAG and forward-backward algorithm, the high-level idea can be described in the following manner:

Step 1. First, the embodiments construct the trellis where the unknown input symbols $M_l$ form a part of the hidden state variables, and where traces $\mathbf{Y}^1$, $\mathbf{Y}^2$, ..., $\mathbf{Y}^K$ are the observables.
Step 2. The embodiments then use the forward-backward algorithm on this trellis to compute the posterior marginal distribution of each state variable.
Step 3. Finally, for each $M_l = m$, the embodiments collect the states which the message is a part of and aggregate their marginal probabilities to obtain $Pr(M_l = m | \mathbf{Y}^1, \mathbf{Y}^2, \dots, \mathbf{Y}^K)$.

Symbolwise Posteriors For One Trace With No Code

To help progressively build an understanding of how the embodiments operate, an example involving the simplest case will first be presented, namely, the example assumes that $\mathbf{M} = \mathbf{X}$. For an input sequence $\mathbf{X} = X_1 X_2 \dots X_N$, where each $X_i$ has a support $\chi$, and given trace $\mathbf{Y} = \mathbf{y}$ of $\mathbf{X}$, it is possible to compute the following in this example case:

$$\Pr(X_i = x | \mathbf{Y} = \mathbf{y}) \; \forall \, i, x$$

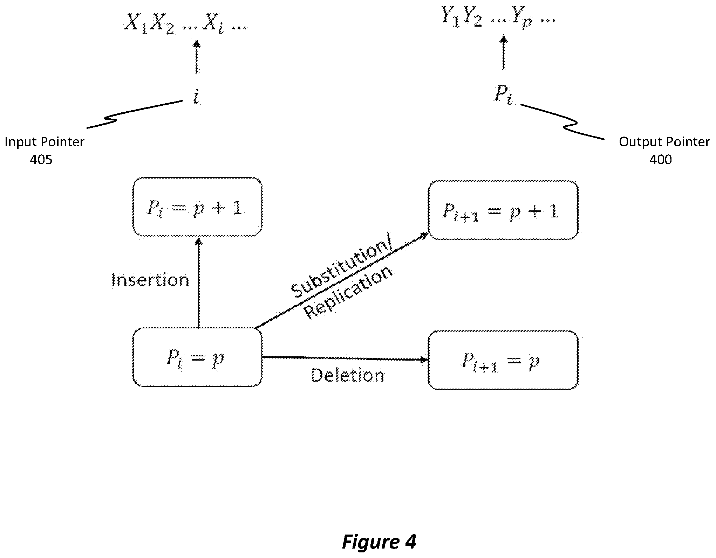

The idea behind constructing the trellis is to model the IDS channel as a FSM by tracking the output pointer $P_i$ (e.g., output pointer 400 as shown in Figure 4) at a given input $X_i$, that determines the index of the next output symbol emitted, as shown in Figure 4. That is, the process of independently constructing any number of trellises can be performed by modelling the IDS channel as a FSM. This construction can also include tracking an output pointer pointing to specific nucleotides included in a particular noisy trace, as shown in Figure 4. Here, the noisy trace is included among the multiple noisy observations 135A (or "traces") of Figure 1A. Performing these operations enables the embodiments to determine an index of a next output nucleotide that is emitted. Optionally, the output pointer can be used to determine a position in the noisy trace where the next output nucleotide is to be appended. The disclosure will now describe the states and edges of the IDS trellis sequentially.

States of the trellis.

It is beneficial to first mention the construction of trellis states, without any reasoning. This idea will become clear when the edges are described.

The state $S_1$ at stage 1 is the output pointer $P_1$ (e.g., output pointer 400) before the first input was received. Suppose $\mathbf{y} = ... y_R$, then the support of $P_i$ is $\mathcal{P} = (1, 2, ..., R + 1)$ for all $i$. The states at stages 2 and 3 are $S_2 = (P_1, X_1)$ and $S_3 = (P_2, X_1)$. The first 3 stages together comprise of one "input cycle." The next 3 states $S_4 = P_2$, $S_5 = (P_2, X_2)$ and $S_6 = (P_3, X_2)$ constitute the second input cycle, and this goes on until all $N$ input symbols are exhausted.

Trellis initialization.

Recall that the trellis is also defined by an initial distribution on $S_1 = P_1$. The output pointer must begin at $P_1 = 1$. Therefore, the embodiments initialize the initial distribution on $S_1$ as $\Pr(S_1 = 1) = 1$ and $\Pr(S_1 = s) = 0$ otherwise.

As described earlier, IDS events may be modeled as a FSM. In Figure 4, the output pointer $P_i$ (i.e. output pointer 400) determines the position in the trace where the next emitted symbol would be appended. Suppose the channel makes an insertion, the input pointer 405 does not change while the output pointer 400 increases by 1. Similarly, a deletion results in no change to the output pointer 400. A substitution/replication results in both the input pointer 405 and the output pointer 400 increasing by 1.

Edges of the trellis.

At this stage, the embodiments can now construct the edges. Constructing these edges will also explain how the trellis models the events in the IDS channel. Three stages to construct the edges will now be described, namely, a stage of modeling the input distribution, a stage of modeling the IDS events, and a stage of transitioning to the next input.

Modeling the input distribution.

The outgoing edges connecting stage 1 and stage 2 model the first input symbol received ($X_1$). For each $p \in \mathcal{P}$ and $x \in \chi$, an unlabeled edge connects vertex $P_1 = p$ in stage 1 to $(P_1, X_1) = (p, x)$ in stage 2; the weight of this edge is $\Pr(X_1 = x)$. Traversing this edge corresponds to the event "the symbol $x$ was input to the IDS channel".

Modeling the IDS events.

The outgoing edges from stage 2 model the events that occur after $X_1 = x$ has been input to the IDS channel. Note that $X_1$ is stored as a part of $S_2$, so the events depend on the value in the input buffer. Consider each vertex $(P_1 = p, X_1 = x)$. It is possible to draw $|\chi|$ intra-stage edges from $(P_1 = p, X_1 = x)$ to $(P_1 = p + 1, X_1 = x)$, each with a unique label $Y_p = y$, where $y \in \chi$ and a weight equal to $\frac{p_{ins}}{|\chi|}$. Each of these edges corresponds to a particular symbol that is inserted. Similarly, it is possible to construct inter-stage edges of appropriate weights to represent substitution, replication, and deletion events.

Transitioning to the next input.

Recall that $S_3 = (P_2, X_1)$. The outgoing edges from stage 2 have taken into account all the events after $X_1$ was input and before $X_2$ is input. The outgoing edges from stage 3 simply remove the input buffer in order to prepare for the next input symbol $X_2$. In other words, an unlabeled edge of weight 1 connects the vertex $(P_2 = p, X_i = x)$ in stage 3 to $P_2 = p$ in stage 4 for all $p, x$.

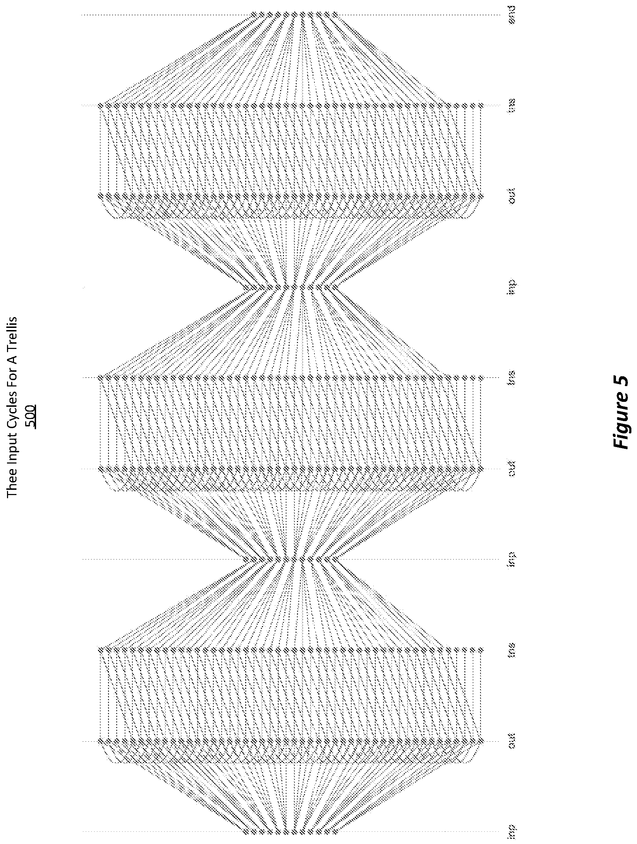

The above three steps model the edges in the first input cycle. These steps are repeated until all the input symbol are accounted for. Figure 5 illustrates 3 input cycles of a trellis 500. Specifically, Figure 5 shows three input cycles in the IDS trellis for a single trace and no code. The arrows on the directional edges and parallel edges have been removed to declutter the graph and for aesthetics. The curved intra-state edges are the edges corresponding to insertion events.

Next the forward-backward algorithm described earlier is used to compute the posterior marginal distributions $\Pr(S_t = s | \mathbf{Y} = \mathbf{y})$, for each stage $t$ and state value $s$.

By way of example, suppose it is desirable to use this to compute $\Pr(X_1 = x | \mathbf{Y} = \mathbf{Y})$, for instance. First, the embodiments note that $X_1$ is a part of the states at stages 2 and 3, namely, $S_2 = (P_1, X_1)$, $S_3 = (P_2, X_1)$. Therefore, to compute the marginal distribution of $X_1$, another round of marginalization over either $S_2$ or $S_3$ is performed, as shown below:

$$\mathbf{Pr}(X_1 = x \mid Y = y) = \sum_p \mathbf{Pr}(S_3 = (p, x) | Y = y)$$

Computing the symbolwise posteriors above involved the three steps of constructing the trellis, running the forward-backward algorithm, and then aggregating the state marginals to compute the input marginals. The first step requires $O(E)$ time and space complexity, where $E$ is the number of edges in the IDS trellis. It is acceptable to assume that the number of vertices is at most the number of edges. The second step runs in $O(E)$ as previously shown. The third step also runs in $O(E)$ as it needs to iterate over the vertices exactly twice, in the worst case scenario. The time complexity of computing the symbolwise posteriors is, therefore, $O(E)$.

One question to ask is: what is $E$ as a function of $N$ (input sequence length) and $R$ (trace length)? It is acceptable to assume that the size of the alphabet $|\chi|$ is a constant. To answer this question, it can first be noted that the number of stages in the trellis is $O(N)$, and at each stage the state-space $S_t$ is either the set of output pointer values $\mathcal{P}$ or is $\mathcal{P} \times \chi$, where $\times$ represents cartesian product of sets. Therefore, $|\boldsymbol{S}_t| = O(|\mathcal{P}|) = O(R)$ for all $t$. Now the max vertex out-degree depends only on $|\chi|$, and is therefore a constant. Thus, the number of edges $E = O(NR)$.

Symbolwise Posteriors For One Trace And With An Encoder

With an understanding gained from that simple example, it is now possible to build on the ideas described so far. For example, consider a message sequence $\mathbf{M} = M_1M_2...M_L$, which is mapped onto a codeword $\mathbf{X} = X_1X_2..X_N$ using a (time-varying) deterministic FSM. A deterministic FSM accepts an input symbol $W$ and outputs a fixed number of symbols $Z_1Z_2..Z_u$, $u \geq 1$. The next state and the output are a deterministic function of the input and current state, contrasting it with a probabilistic FSM. However, it is acceptable to allow the deterministic function and the output length $u$ itself to vary with time. [00152] Assume that the support of each $M_i$ is M, and the support of $X_i$ is $\chi$, where the size of the alphabets $|\mathcal{M}|$ and $|\chi|$ is a constant. Suppose the trace $\mathbf{Y} = \mathbf{y}$ of $\mathbf{X}$ is observed. As performed earlier, let $y = y_1y_2...y_R$. In this next section, the disclosure will describe the computation of the symbolwise posterior, as shown below:

$$\mathbf{Pr}(M_l = m | Y = y) \ \forall \ l, m$$

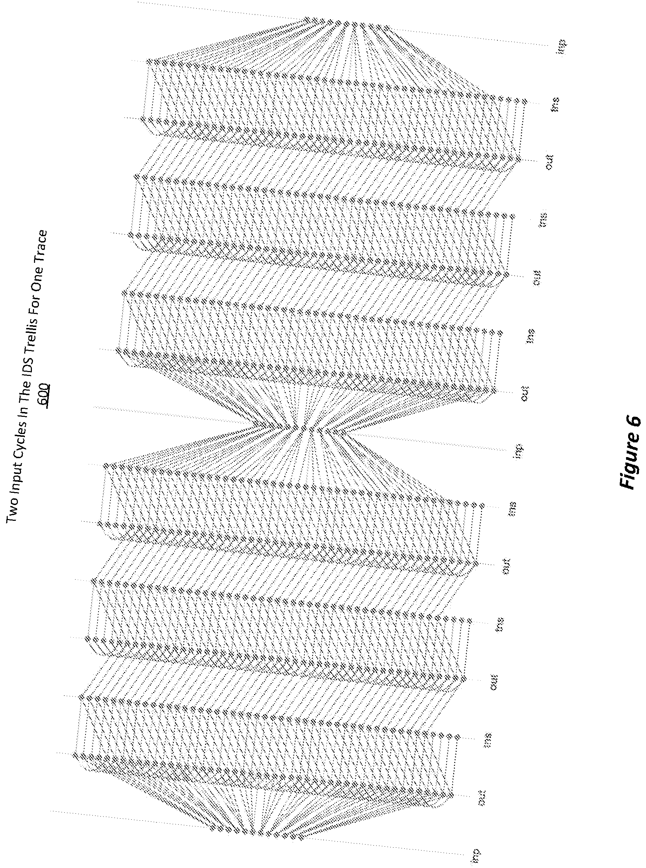

Constructing the IDS trellis in this scenario is performed by simultaneously tracking the state of the encoder and the output pointer. For a given input symbol, the encoder outputs $u \geq 1$ output symbols. In the IDS trellis, the IDS channel events corresponding to these output symbols are modeled one at a time. Figure 6 is an example of such a trellis with a rate 1/3 encoder.

In particular, Figure 6 shows an example of two input cycles in the IDS trellis for one trace 600, where the codewords are produced by a (possibly time-varying) rate 1/3 ($u = 3$) encoder. The arrows on the directional edges and parallel edges have been removed in Figure 6 to declutter the graph and for aesthetics.

Constructing this trellis will now be described in four stages. The first stage involves modeling the input. The second stage involves modeling the IDS events. The third stage involves updating the output buffer. The final stage involves transitioning to the next input.

Modeling The Input

At this first stage, the IDS trellis state is $S_1 = (Q_1, P_1)$, the joint initial state of the encoder and output pointer. Now, the encoder at the current state $Q_1$ receives the input symbol $M_1$, emits $u$ output symbols $X_1X_2...X_u$ and transits to the next state $Q_2$. It is possible to divide this into u stages, one for each codeword symbol. Therefore, the state at stage 2 in the trellis can be modeled to be $S_2 = (Q_1, P_1, M_1, X_1)$. An edge of weight $Pr(M_1 = m)$ connects $(Q_1 = q, P_1 = p)$ with $(Q_1 = q, P_1 = p, M_1 = m, X_1 = x)$ for each $p, m, q$. Note that fixing $m$ and $q$ automatically fixes $x$.

Modeling the IDS events.

The outgoing edges from stage 2 model the IDS channel events after the first codeword symbol $X_1 = x_1$ has been input to the IDS channel. The trellis state at stage 3 is $S_3 = (Q_1 = q, P_2 = p', M_1 = m, X_1 = x)$. Here, only the output pointer has been updated from stage 2 to stage 3.

Updating the output buffer.

Next, the embodiments can update the output buffer to replace $X_1$ with $X_2$. Again, $X_2$ is a deterministic function of the encoder state $Q_1$ and input symbol in the buffer $M_1$, hence there are unlabeled edges of weight 1 connecting state ($Q_1 = q, P_2 = p, M_1 = m, X_1 = x$) at stage 3 to state ($Q_1 = q, P_2 = p, M_1 = m, X_2 = x'$) at stage 4, where $x'$ is determined by the choices of $q$ and $m$.

Transitioning to the next input.

The above two steps of modeling the IDS events and updating the output buffer are repeated until all $u$ codeword symbols are accounted for. Finally, the input and output buffers are cleared and the encoder transitions to its new state $Q_2$ to prepare for the next input symbol $M_2$.

The above steps are performed for one input cycle. These steps are repeated until all input symbols are accounted for. Figure 6 shows an example of such a trellis with a rate $1/3$ ($u = 3$) encoder illustrated for 2 input cycles. With this description, the posterior probability computation follows as detailed earlier (i.e. compute the posterior marginals over the trellis states and marginalize it further to obtain Pr($M_1 = m$ | $\mathbf{Y} = \mathbf{y}$)). The time complexity to compute the symbolwise posteriors is $O(NR|\mathcal{Q}|)$, where $\mathcal{Q}$ is the state-space of the encoder FSM, as the number of vertices have increased by a factor of $|\mathcal{Q}|$.

Symbolwise Posteriors For Multiple Traces And With An Encoder

With the understanding gained from the previous two examples, the disclosure will now further build on those concepts in order to construct the "multi-trace IDS trellis" that accommodates multiple traces simultaneously. The setup is the same as described in the previous example, except that now $K$ traces $\mathbf{Y}^l = \mathbf{y}^1$, ..., $\mathbf{Y}^K = \mathbf{y}^K$ of $\mathbf{X}$ are observed instead of one trace. Let $y^k = y_1^k y_2^k \cdots y_{R_k}^k$. The desire is to compute the following:

$$\mathbf{Pr}(M_l = m \mid Y^1 = y^1, ..., Y^K = y^K) \forall \, l, m$$

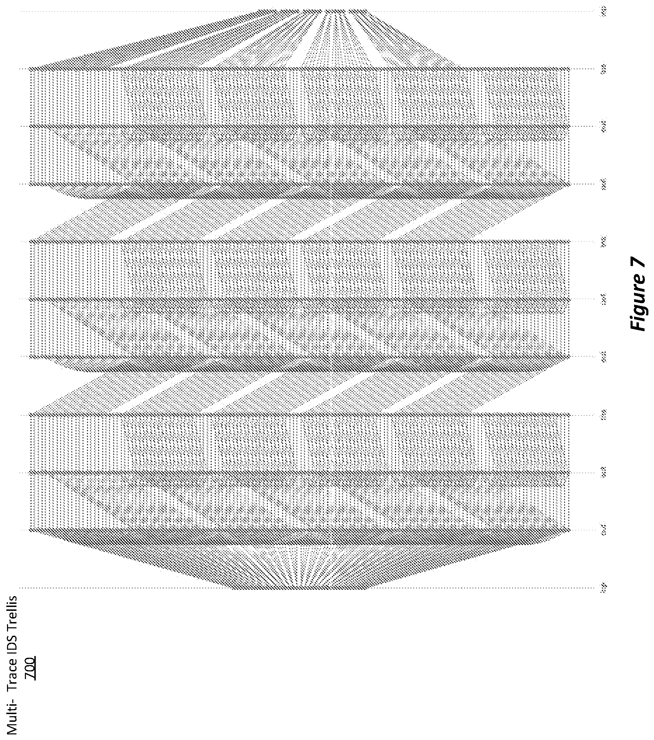

Here, there are K different output pointers assigned, each corresponding to one trace. Therefore, the trellis is built to simultaneously track the state of the encoder and the $K$ output pointers. Moreover, a notable detail in the construction that avoids local exponential blowup in the number of edges is that the IDS channel events for the traces are modeled sequentially. In other words, $K$ successive stages are constructed, where the outgoing edges from each stage model the IDS channel events for exactly one trace. Figure 7 shows an example of a multi-trace IDS trellis 700 generated in accordance with the principles that will be discussed below.

Specifically, the multi-trace IDS trellis 700 is for 2 traces used with a rate 1/3 encoder. In other words, this single trellis is a combined trellis that includes data for multiple traces (in this case 2). It is preferable to have smaller sized trellises because of the potential for exponential growth when multiple traces are combined. That said, the embodiments can impose a threshold number when determining whether to combine the data for multiple traces. For instance, the threshold number may indicate that a maximum of 2 traces can be combined in a single trellis. Of course, other numbers can be used. Therefore, if the number of traces is less than the threshold number, then a single trellis can be generated based on those traces. Otherwise, smaller trellises are preferred, where it is likely that a single trellis will be generated for a single trace. As such, Figure 7 illustrates one possible scenario of a trellis, but this possible scenario is more likely an outlier scenario.

There are four stages used to construct the multi-trace IDS trellis. One stage involves modeling the input. Another stage involves modeling the IDS events. Another stage involves updating the output buffer. Another stage involves transitioning to the next input.

Modeling the input.

At stage 1, the trellis state is $S_1 = (Q_1, P_1^1, P_1^2, ..., P_1^K)$, the joint initial state of the encoder and output pointers. The state at stage 2 appends to the previous state the first input symbol and the first codeword symbol emitted by the encoder (i.e. $S_2 = (Q_1, P_1^1, P_1^2, ..., P_1^K, M_1, X_1)$) and corresponding edges from stage 1 to 2 model the input distribution.

Modeling the IDS events.

The next stage is $S_3 = (Q_1, P_2^1, P_1^2, \ldots, P_1^K, M_1, X_1)$. Note that only the output pointer corresponding to trace 1 ($P^1$) has been updated from stage 2 to stage 3; the outgoing edges from stage 2 model the IDS events with $X_1$ in trace 1. Next, the outgoing edges from stage 3 model the IDS events with $X_1$ in trace 2, so that $P^2$ is updated. This is repeated until all $K$ traces are exhausted.

Updating the output buffer.

Next, the embodiments update the output buffer to replace $X_1$ with $X_2$. Again, $X_2$ is a deterministic function of the encoder state $Q_1$ and input symbol in the buffer $M_1$. This is followed by $K$ stages of IDS event modeling for $X_2$.

Transitioning to the next input.

The above two steps of modeling the IDS events and updating the output buffer are repeated until all codeword symbols corresponding to a given input symbol are accounted for. Finally, the input and output buffer are cleared and the encoder transitions to its new state $Q_2$ to prepare for the next input symbol $M_2$.

The above steps are performed for one input cycle. These steps are repeated until all input symbols are accounted for. The multi-trace IDS trellis 700 of Figure 7 illustrates an example of a multi-trace IDS trellis for 2 traces with a rate 1/3 ($u = 3$) encoder illustrated for 1 input cycle.

In particular, the multi-trace IDS trellis of Figure 7 provides an example of 1 input cycle in the multi-trace IDS trellis for 2 traces and rate 1/3 encoder. The arrows on the directional edges and parallel edges have been removed to declutter the graph and for aesthetics. The first stage models the input and appends the first codeword to the output buffer. The trellis next models all possible events with the first codeword symbol in the first trace. Then, the trellis models events in the second trace. Next, the trellis replaces the codeword symbol in the output buffer and models the IDS events with the second codeword symbol in the two traces. Finally, the trellis models the IDS events with the third codeword symbol in the two traces before transitioning to the next input symbol.

With that description, the posterior probability computation follows as detailed earlier (i.e. compute the posterior marginals over the trellis states and marginalize it further to obtain $Pr(M_l = m \mid \mathbf{Y}^1 = \mathbf{y}^1, \ldots, \mathbf{Y}^K = \mathbf{y}^K) \; \forall \; l, m$.

The number of vertices in each stage of the trellis is $O\left(|Q|\prod_{k=1}^{K} R_k\right)$, where $Q$ is the statespace of the encoder FSM and $R_k$ is the length of $k^{th}$ trace observed $\mathbf{y}_k$. The time complexity to compute the symbolwise posteriors is, therefore, $O\left(N||Q|\prod_{k=1}^{K} R_k\right)$ as the out-degree of each vertex is a constant.

It is often convenient in practice to assume that in the IDS channel, the output pointer does not "drift" very far away from the input pointer. More precisely, the output pointer $P_i$ is assumed to lie in the set $\mathcal{P} = \left\{ i - \frac{D}{2}, i - \frac{D}{2} + 1, \ldots, i, \ldots, i + \frac{D}{2} \right\}$, where $D$ is called the "max drift." This helps limit the size of trellis state-space at each stage.

With this assumption, the time complexity in computing the symbolwise posteriors becomes $O(N D^K |Q|)$, where $N$ is the codeword length, $D$ is max drift, and $Q$ is the state-space of the FSM encoder. It is also useful to note that the trellis can be constructed once, given the drift value, and be reused to compute symbolwise posteriors with multiple instances of observed traces.

Trellis BMA: A Heuristic For Coded TR

Given the exponential growth of the multi-trace IDS trellis with the number of traces, it is beneficial to next describe a heuristic that uses the smaller IDS trellises built with each individual traces to construct an estimate $\widehat{M_1} \, \widehat{M_2} \ldots \widehat{M_I}$ of the message symbols sequentially. Such a process can be performed by aggregating the beliefs $Pr(M_i \mid \mathbf{Y} = \mathbf{y}^k)$ obtained from each individual trace $\mathbf{y}^k$.

The process starts by first describing the goal: given $K$ traces $\mathbf{Y}^1 = \mathbf{y}^1, \ldots, \mathbf{Y}^K = \mathbf{y}^K$, construct an estimate

$\widehat{M_1}\ \widehat{M_2}\ ...\ \widehat{M_I}$ of the message sequence. The trellis BMA will now be described.

During an initialization stage, the embodiments first construct $K$ trellises independently using each trace $\mathbf{y}^k$, following the steps outlined in the earlier description (e.g., "Symbolwise Posteriors For One Trace And With An Encoder"). Next, the embodiments run the forward-backward algorithm on each of the $K$ trellises with the corresponding traces as observations. Let $F^k(v)$ and $B^k(v)$ denote the forward and backward values of a vertex $v$ in the trellis corresponding to trace $k$; these values will be updated as the algorithm is run.

During a decoding stage, the embodiments now compute each $\widehat{M_I}$ by iterating through the following two steps. Assume that $M_1, M_2 ... M_{I-1}$ has already been computed and that it is desired to compute $\widehat{M_I}$ .

First, there is a process of combining beliefs to make a hard decision. To do so, the embodiments use the current values of $F^k(v)$ and $B^k(v)$ to compute each trellis' current "belief" about symbol $M_l$, denoted by $V^k(M_l = m)$, as follows. First, recall that each $M_l$ is a part of the trellis state in the stages corresponding to input cycle $l$. Pick one such stage $t$ (the last stage of the input cycle), and define the following:

$$V^k(M_l = m) \triangleq \sum_v F^k(v)B^k(v)$$

where the summation is over all vertices in the stage where $M_l = m$. Turning briefly to Figure 3, this figure shows an example of the probability-based beliefs 325. Next, a hard decision is made on each input symbol by aggregating the beliefs. In other words, the embodiments assign:

$$\widehat{M_l} \leftarrow \arg max_m \sum_k V^k(M_l = m)$$

Next, there is a phase of updating the forward values. Specifically, the embodiments go back to stage $t$ picked above and update the forward values for vertices in this stage. To do this, the embodiments pick vertices v which disagree with the hard-decision on the current symbol, i.e., vertices where $M_l \neq \widehat{M_l}$ . For every such vertex in each of the $K$ trellises, the forward values are updated as follows:

$$F^k(v) \leftarrow \epsilon F^k(v), \forall\, k, \text{ and } \forall\, v \text{ where } M_l \neq \widehat{M_l}$$

where $\varepsilon < 1$ is a small non-negative constant. In the numerics, $\varepsilon = 0.1$ worked well. The forward values are then normalized across the stage.

Using the updated forward values at input cycle $l$, the embodiments then perform a forward pass (using the update rule given by (6)) over the next input cycle $l + 1$ to recompute the forward values corresponding to the vertices of this input cycle. These values are then used for making a decision on $M_{l+1}$.

The idea of updating the forward values sequentially computes the estimates $\widehat{M_1}\ \widehat{M_2}\ ...\ \widehat{M_I}$ . Analogously, one could start from the end of the trellis and update the backward values to compute an estimate $\widetilde{M_1}\widetilde{M_2}\ ...\widetilde{M_3}$ which proceeds in the reverse order. The embodiments can then take the first half from the forward estimate and second half from the reverse estimate to construct a new estimate $\widehat{M_1}\ \widehat{M_2}\ ...\ \widehat{M_{\frac{I}{2}}}\ \widetilde{M_{\frac{I}{2}+1}}...\widetilde{M_I}$ of the input.

To determine the time complexity, let $D$ be the max drift value for the output pointers, and $N$ be the codeword length, and $|Q|$ be the size of encoder state-space. The initialization step involves constructing the $K$ trellises and running forward-backward algorithm on each of these. This takes $O(K N D |Q|)$ time. The next step of decoding iterates over the vertices of each trellis thrice (once to compute the beliefs, twice to update and normalize the forward values) and over the edges of each trellis once (to redo the forward pass). Therefore, this step takes $O(K N D |Q|)$ time as well, and thus trellis BMA runs in $O(K N D | Q |)$.

Performance

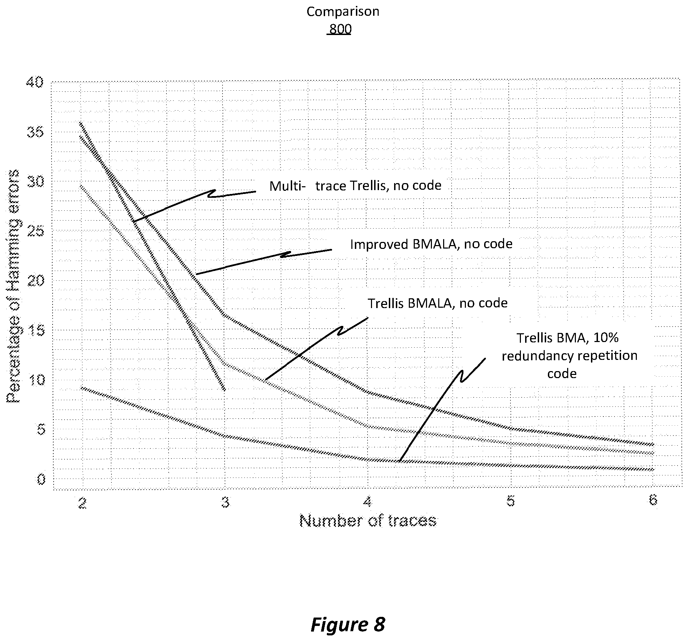

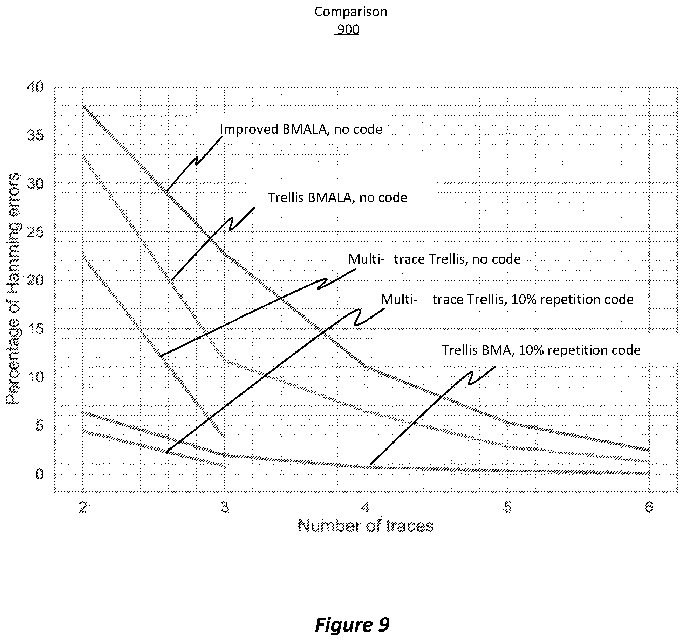

To better understand the performance improvements provided by the disclosed embodiments, this section will now compare the performance of the two proposed approaches to TR and coded TR (symbolwise MAP on multi-trace trellis and trellis BMA) to the state-of-the-art TR heuristic currently used (improved BMALA). On real data, as shown by the comparison 800 chart in Figure 8, even when used as standalone TR algorithms, both the "multi-trace trellis" and "trellis BMA" approaches improve upon the current state-of-the-art in the interest of regime ($\geq$ 3 traces). Moreover, as seen from in the comparison 800 of Figure 8 and the comparison 900 of Figure 9, using a low-redundancy code (10% redundancy here) provides significant improvement in performance.

In particular Figure 8 shows the performance on real data. More particularly, error rates on real data from Nanopore sequencing is illustrated, where there is comparison between the algorithms described herein (e.g., symbolwise MAP on multi-trace trellis and trellis BMA) to the previous state-of-the-art TR algorithm (improved BMALA).

Figure 9 shows the performance on simulated data. Here, error rates on simulated data (e.g., where the IDS errors have been simulated) are used to compare the algorithms described herein (e.g., symbolwise MAP on multi-trace trellis and trellis BMA) to the previous state-of-the-art TR algorithm (improved BMALA).

Symbolwise MAP Minimizes Expected Hamming Distance

Consider a channel which accepts an input sequence $\mathbf{X} = X_1 X_2.., X_N$ and outputs a set of observations $\mathbf{Y}$ ($\mathbf{Y}$ can be multiple sequences as well). Suppose it is desirable to compute an estimate of $\mathbf{X}$ from $\mathbf{Y}$ (call it $\hat{\mathbf{X}}(\mathbf{Y})$). For simplicity, assume that $\mathbf{X}$ is a binary sequence, although the following arguments extend to arbitrary alphabets.

Symbolwise MAP is an optimal estimator for minimizing the expected Hamming distance between $\mathbf{X}$ and $\hat{\mathbf{X}}$, for any channel regardless of whether it is memoryless or not. This fact can be seen from the following argument. Suppose $\mathbf{Y} = \mathbf{y}$ is observed. The expected Hamming distance of an estimator, given $\mathbf{Y} = \mathbf{y}$ is the following:

$$\sum_{i=1}^{N} \mathbf{Pr}\big(X_i \neq \widehat{X_i}(y)\big|Y = y\big)$$

$$= \sum_{i=1}^{N}\big(\mathbf{Pr}(X_i = 0|Y = y)\,\mathbf{Pr}\big(\widehat{X_i}(y) = 1\big|X_i = 0, Y = y\big)$$

$$+ Pr(X_i = 1|Y = y)\mathbf{Pr}(\widehat{X_i}(y) = 0|X_i = 1, Y = y)\big)$$

However, the Markov chain $X_i \to Y \to \widehat{X_i}$ and hence $\widehat{X_i}$ is conditionally independent of $X_i$ given $\mathbf{Y}$, which implies the following:

$$\sum_{i=1}^{N} \mathbf{Pr}\big(X_i \neq \widehat{X_i}(y)\big|Y = y\big)$$

$$= \sum_{i=1}^{N}\big(\mathbf{Pr}(X_i = 0|Y = y)\,\mathbf{Pr}\big(\widehat{X_i}(y) = 1\big| Y = y\big)$$

$$+ Pr(X_i = 1|Y = y)\mathbf{Pr}(\widehat{X_i}(y) = 0| Y = y)\big)$$

To simplify notation, let the posterior probabilities be $q_i(y) \triangleq \mathbf{Pr}(X_i = 1|Y = y)$ and let $\alpha_i(y) \triangleq \mathbf{Pr}(\widehat{X_i}(y) = 1|Y = y)$. Note that $q_i(\mathbf{y})$ is a property of the channel and is fixed given $\mathbf{y}$, while $\alpha_i(\mathbf{y})$ depends on the estimator. With this, the above expression can be re-written as follows:

$$\sum_{i=1}^{N} \Pr(X_i \neq \widehat{X_i}(y) | Y = y) = \sum_{i=1}^{N} \Big( \big(1 - q_i(y)\big)\alpha_i(y) + q_i(y)(1 - \alpha_i(y)) \Big)$$

$$\geq \sum_{i=1}^{N} min\{(1 - q_i(y)), q_i(y)\}$$

The optimal assignment of $\alpha_i(\mathbf{y})$ that satisfies the lower bound with equality is $\alpha_i(\mathbf{y}) = 1$ if $q_i(\mathbf{y}) \geq 0.5$ and $\alpha_i(\mathbf{y}) = 0$ otherwise, which coincides with the symbolwise MAP estimate.

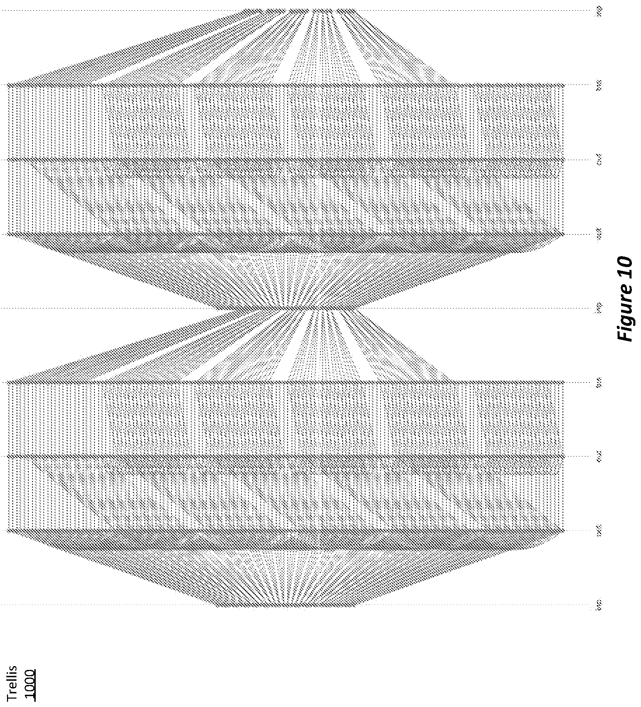

Figure 10 illustrates another trellis 1000 providing an example of 2 input cycles in the IDS trellis for 2 traces and no code. The arrows on the directional edges and parallel edges have been removed to declutter the graph and for aesthetics. Each input cycle first models the input marginal distribution. The trellis 1000 next models all possible events in the first trace and then models events in the second trace. Finally, the trellis 1000 transits to the next input cycle.

Example Methods

The following discussion now refers to a number of methods and method acts that may be performed. Although the method acts may be discussed in a certain order or illustrated in a flow chart as occurring in a particular order, no particular ordering is required unless specifically stated, or required because an act is dependent on another act being completed prior to the act being performed.

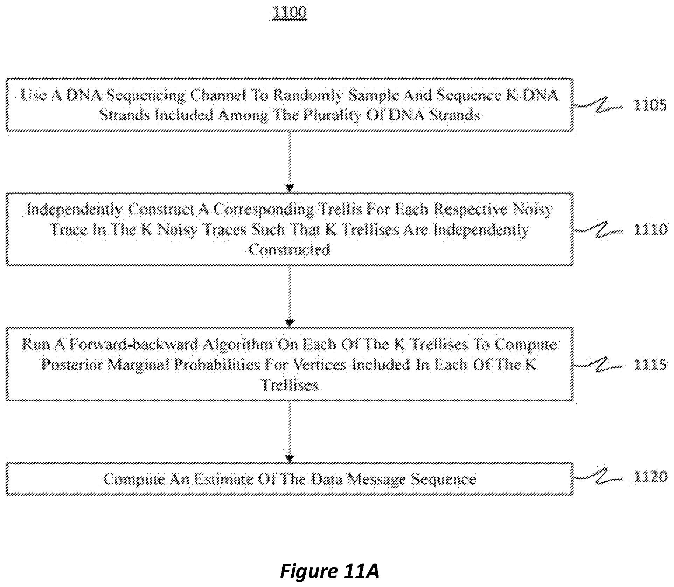

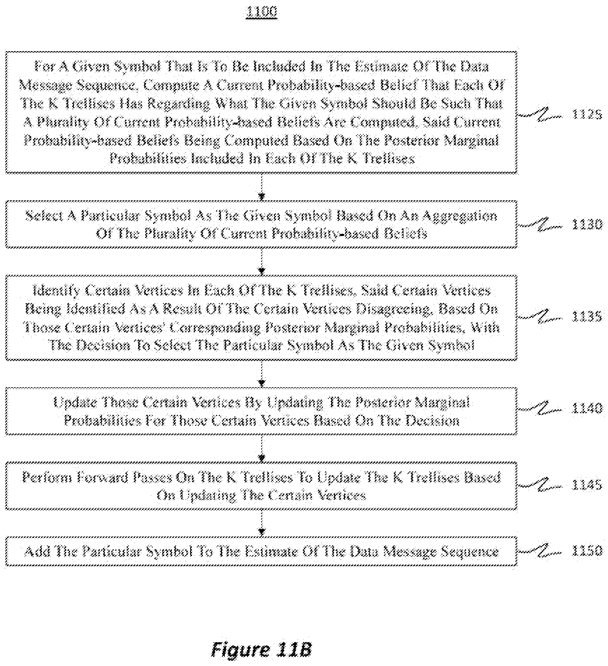

Figures 11A and 11B illustrate a flowchart of an example method 1100 configured to facilitate reductions in cost of encoding and decoding operations used in deoxyribonucleic acid (DNA) data storage systems. Method 1100 is further configured to facilitate reducing errors in the encoding and decoding operations while accounting for a code structure used during the encoding and decoding by constructing and using insertion-deletion-substitution (IDS) trellises for multiple traces.

Method 1100 begins after a data message sequence, which includes multiple symbols (e.g., binary symbols, such as zeros and ones), has been encoded into a DNA sequence and after the DNA sequence has been synthesized into multiple DNA strands (e.g., perhaps thousands or even millions). After those operations, act 1105 involves using a DNA sequencing channel to randomly sample and sequence K (i.e. a number greater than one) DNA strands included among the multiple DNA strands. Here, the sequencing process results in a generation of K noisy traces of the DNA sequence. Furthermore, the DNA sequencing channel is modeled as a simplistic zeroth order approximation in a form of an IDS channel.

Act 1110 then involves independently constructing a corresponding trellis for each respective noisy trace in the K noisy traces. As a consequence, K trellises are independently constructed. Notably, the IDS channel is modeled as a finite state machine (FSM) to construct each of the K trellises as has been discussed in this disclosure. Optionally, at least one of the trellises is prepended with a dummy stage having one vertex structured to disallow intra-stage edges at that trellis's first stage.

As shown in Figure 3, one of the trellises (e.g., a "first" trellis) includes a first set of vertices and edges between pairs of vertices in the first set of vertices. Here, every edge in the first trellis is weighted, and a sum of all weights for all edges of any particular vertex in the first trellis has a value of one. In some cases, the first trellis can be configured so that no path spanning multiple vertices in the first trellis contains two edges corresponding to two realizations of a same observable. Furthermore, as has been discussed in detail, the first trellis can induce a joint distribution, thereby causing the first trellis to be a Hidden Markov Model.

Act 1115 includes running a forward-backward algorithm (e.g., forward-backward algorithm 320 from Figure 3) on each of the K trellises to compute posterior marginal probabilities for vertices included in each of the K trellises. As discussed earlier, the process of running the forward-backward algorithm can include a number of steps. These steps include modifying the K trellises to explain a set of observations. The steps also include computing forward values for each vertex in each of the K trellises. Additionally, the steps include computing backward values for each vertex in each of the K trellises and also computing the posterior marginal probabilities for the vertices in each of the K trellises using the forward values and the backward values. In some cases, the process of computing the forward values includes computing topological orderings for the vertices of the K trellises. When computing the backward values, a reverse of the topological orderings can be used for the vertices of the K trellises.

Act 1120 then involves computing an estimate of the data message sequence by iterating through the steps outlined in Figure 1100B until all estimated symbols forming the estimate of the data message sequence are determined.

The subprocesses or steps of act 1120 are outlined in Figure 1100B. Specifically, act 1125 is performed for a given symbol that is to be included in the estimate of the data message sequence. Act 1125 involves computing a current probability-based belief that each of the K trellises has regarding what the given symbol should be such that multiple current probability-based beliefs are computed. These current probability-based beliefs are computed based on the posterior marginal probabilities included in each of the K trellises.

Act 1130 then involves selecting a particular symbol as the given symbol based on an aggregation of the current probability-based beliefs. For example, this selection may occur by selecting whichever vertex has the highest probability.

Act 1135 includes identifying certain vertices in each of the K trellises. These certain vertices are identified as a result of the certain vertices disagreeing, based on those certain vertices' corresponding posterior marginal probabilities, with the decision to select the particular symbol as the given symbol.

In act 1140, those certain vertices are updated by updating the posterior marginal probabilities for the certain vertices based on the decision. Act 1145 then includes performing forward passes on the K trellises to update the K trellises based on updating the certain vertices.

Act 1150 then involves adding the particular symbol to the estimate of the data message sequence. Optionally, a number of symbol mismatches between symbols included in the original data message sequence and symbols included in the estimate of the data message sequence can be minimized by reducing the Hamming distance between the data message sequence and the estimate of the data message sequence. All of these different acts have been discussed in detail throughout the earlier portions of this disclosure.

Example Computer / Computer Systems

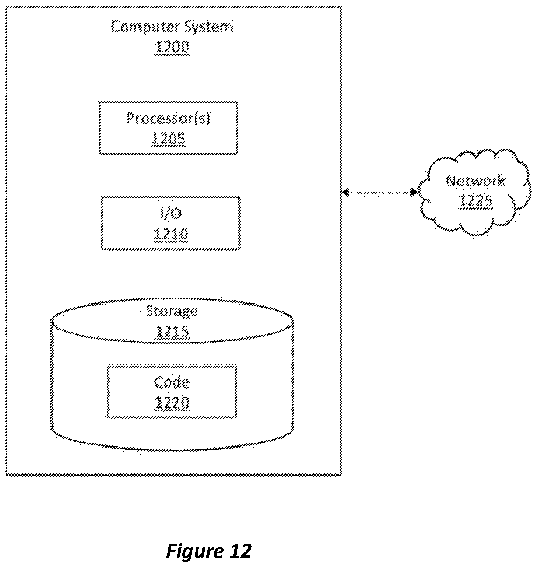

Attention will now be directed to Figure 12 which illustrates an example computer system 1200 that may include and/or be used to perform any of the operations described herein. Computer system 1200 may take various different forms. For example, computer system 1200 may be embodied as a tablet, a desktop, a laptop, a mobile device, or a standalone device. Computer system 1200 may also be a distributed system that includes one or more connected computing components/devices that are in communication with computer system 1200.

In its most basic configuration, computer system 1200 includes various different components. Figure 12 shows that computer system 1200 includes one or more processor(s) 1205 (aka a "hardware processing unit"), input/output I/O 1210, and storage 1215.

Regarding the processor(s) 1205, it will be appreciated that the functionality described herein can be performed, at least in part, by one or more hardware logic components (e.g., the processor(s) 1205). For example, and without limitation, illustrative types of hardware logic components/processors that can be used include Field-Programmable Gate Arrays ("FPGA"), Program-Specific or Application-Specific Integrated Circuits ("ASIC"), Program-Specific Standard Products ("ASSP"), System-On-A-Chip Systems ("SOC"), Complex Programmable Logic Devices ("CPLD"), Central Processing Units ("CPU"), Graphical Processing Units ("GPU"), or any other type of programmable hardware.

As used herein, the terms "executable module," "executable component," "component," "module," or "engine" can refer to hardware processing units or to software objects, routines, or methods that may be executed on computer system 1200. The different components, modules, engines, and services described herein may be implemented as objects or processors that execute on computer system 1200 (e.g. as separate threads).

The I/O 1210 may include any type of input or output device. Examples include a mouse, screen, keyboard and so forth. An encoder, DNA synthesizer, DNA sequencer, and decoder can also be included among the I/O 1210. The encoder and decoder and be implemented via the processor 1205.

Storage 1215 may be physical system memory, which may be volatile, non-volatile, or some combination of the two. The term "memory" may also be used herein to refer to non-volatile mass storage such as physical storage media. If computer system 1200 is distributed, the processing, memory, and/or storage capability may be distributed as well.

Storage 1215 is shown as including executable instructions (i.e. code 1220). The executable instructions represent instructions that are executable by the processor(s) 1205 of computer system 1200 to perform the disclosed operations, such as those described in the various methods.

The disclosed embodiments may comprise or utilize a special-purpose or general-purpose computer including computer hardware, such as, for example, one or more processors (such as processor(s) 1205) and system memory (such as storage 1215), as discussed in greater detail below. Embodiments also include physical and other computer-readable media for carrying or storing computer-executable instructions and/or data structures. Such computer-readable media can be any available media that can be accessed by a general-purpose or special-purpose computer system. Computer-readable media that store computer-executable instructions in the form of data are "physical computer storage media" or a "hardware storage device." Computer-readable media that carry computer-executable instructions are "transmission media." Thus, by way of example and not limitation, the current embodiments can comprise at least two distinctly different kinds of computer-readable media: computer storage media and transmission media.

Computer storage media (aka "hardware storage device") are computer-readable hardware storage devices, such as RAM, ROM, EEPROM, CD-ROM, solid state drives ("SSD") that are based on RAM, Flash memory, phase-change memory ("PCM"), or other types of memory, or other optical disk storage, magnetic disk storage or other magnetic storage devices, or any other medium that can be used to store desired program code means in the form of computer-executable instructions, data, or data structures and that can be accessed by a general-purpose or special-purpose computer.

Computer system 1200 may also be connected (via a wired or wireless connection) to external sensors (e.g., one or more remote cameras) or devices via a network 1225. For example, computer system 1200 can communicate with any number devices or cloud services to obtain or process data. In some cases, network 1225 may itself be a cloud network. Furthermore, computer system 1200 may also be connected through one or more wired or wireless networks 1225 to remote/separate computer systems(s) that are configured to perform any of the processing described with regard to computer system 1200.

A "network," like network 1225, is defined as one or more data links and/or data switches that enable the transport of electronic data between computer systems, modules, and/or other electronic devices. When information is transferred, or provided, over a network (either hardwired, wireless, or a combination of hardwired and wireless) to a computer, the computer properly views the connection as a transmission medium. Computer system 1200 will include one or more communication channels that are used to communicate with the network 1225. Transmissions media include a network that can be used to carry data or desired program code means in the form of computer-executable instructions or in the form of data structures. Further, these computer-executable instructions can be accessed by a general-purpose or special-purpose computer. Combinations of the above should also be included within the scope of computer-readable media.

Upon reaching various computer system components, program code means in the form of computer-executable instructions or data structures can be transferred automatically from transmission media to computer storage media (or vice versa). For example, computer-executable instructions or data structures received over a network or data link can be buffered in RAM within a network interface module (e.g., a network interface card or "NIC") and then eventually transferred to computer system RAM and/or to less volatile computer storage media at a computer system. Thus, it should be understood that computer storage media can be included in computer system components that also (or even primarily) utilize transmission media.

Computer-executable (or computer-interpretable) instructions comprise, for example, instructions that cause a general-purpose computer, special-purpose computer, or special-purpose processing device to perform a certain function or group of functions. The computer-executable instructions may be, for example, binaries, intermediate format instructions such as assembly language, or even source code. Although the subject matter has been described in language specific to structural features and/or methodological acts, it is to be understood that the subject matter defined in the appended claims is not necessarily limited to the described features or acts described above. Rather, the described features and acts are disclosed as example forms of implementing the claims.

Those skilled in the art will appreciate that the embodiments may be practiced in network computing environments with many types of computer system configurations, including personal computers, desktop computers, laptop computers, message processors, hand-held devices, multi-processor systems, microprocessor-based or programmable consumer electronics, network PCs, minicomputers, mainframe computers, mobile telephones, PDAs, pagers, routers, switches, and the like. The embodiments may also be practiced in distributed system environments where local and remote computer systems that are linked (either by hardwired data links, wireless data links, or by a combination of hardwired and wireless data links) through a network each perform tasks (e.g. cloud computing, cloud services and the like). In a distributed system environment, program modules may be located in both local and remote memory storage devices.

The present invention may be embodied in other specific forms without departing from its characteristics. The described embodiments are to be considered in all respects only as illustrative and not restrictive. The scope of the invention is, therefore, indicated by the appended claims rather than by the foregoing description. All changes which come within the meaning of the claims are to be embraced within their scope.

**Claims**

1. A computer system (1200) configured to facilitate reductions in cost of encoding and decoding operations used in deoxyribonucleic acid, DNA, data storage systems and to facilitate reducing errors in said encoding and decoding operations while accounting for a code structure used during said encoding and decoding by constructing and using insertion-deletion-substitution, IDS, trellises for multiple traces, said computer system comprising:

   one or more processors (1205); and
   one or more computer-readable hardware storage devices (1215) that store instructions that are executable by the one or more processors to cause the computer system to at least:

after a data message sequence, which includes a plurality of symbols, has been encoded into a DNA sequence and after the DNA sequence has been synthesized into a plurality of DNA strands, use (1105) a DNA sequencing channel to randomly sample and sequence K DNA strands included among the plurality of DNA strands, wherein:

said sequencing results in a generation of K noisy traces of the DNA sequence, wherein K is a number greater than 1, and
the DNA sequencing channel is modeled as a simplistic zeroth order approximation in a form of an IDS channel;

independently construct (1110) a corresponding trellis for each respective noisy trace in the K noisy traces such that K trellises are independently constructed, wherein the IDS channel is modeled as a finite state machine, FSM, to construct each of the K trellises;
run (1115) a forward-backward algorithm on each of the K trellises to compute posterior marginal probabilities for vertices included in each of the K trellises;
compute (1120) an estimate of the data message sequence by iterating through the following steps until all estimated symbols forming the estimate of the data message sequence are determined:

for a given symbol that is to be included in the estimate of the data message sequence, compute a current probability-based belief that each of the K trellises has regarding what the given symbol should be such that a plurality of current probability-based beliefs are computed, said current probability-based beliefs being computed based on the posterior marginal probabilities included in each of the K trellises;
select a particular symbol as the given symbol based on an aggregation of the plurality of current probability-based beliefs;
identify certain vertices in each of the K trellises, said certain vertices being identified as a result of the certain vertices disagreeing, based on those certain vertices' corresponding posterior marginal probabilities, with the decision to select the particular symbol as the given symbol;
update those certain vertices by updating the posterior marginal probabilities for those certain vertices based on the decision;
perform forward passes on the K trellises to update the K trellises based on updating the certain vertices; and
add the particular symbol to the estimate of the data message sequence.

2. The computer system of claim 1, wherein a number of symbol mismatches between symbols included in the data message sequence and symbols included in the estimate of the data message sequence is minimized by reducing a Hamming distance between the data message sequence and the estimate of the data message sequence.

3. The computer system of claim 1, wherein at least one trellis in the K trellises is prepended with a dummy stage having one vertex structured to disallow intra-stage edges at a first stage of the at least one trellis.

4. The computer system of claim 1, wherein a first trellis included among the K trellises includes a first set of vertices and edges between pairs of vertices in the first set of vertices,

wherein every edge in the first trellis is weighted, and
wherein a sum of all weights for all edges of any particular vertex in the first trellis has a value of one.

5. The computer system of claim 4, wherein no path spanning multiple vertices in the first trellis contains two edges corresponding to two realizations of a same observable.

6. The computer system of claim 4, wherein the first trellis induces a joint distribution such that the first trellis is a Hidden Markov Model.

7. The computer system of claim 1, wherein running the forward-backward algorithm includes:

modifying the K trellises to explain a set of observations;
computing forward values for each vertex in each of the K trellises;
computing backward values for each vertex in each of the K trellises; and
computing the posterior marginal probabilities for the vertices in each of the K trellises using the forward values and the backward values.

8. The computer system of claim 7, wherein computing the forward values includes computing topological orderings for the vertices of the K trellises.

9. The computer system of claim 8, wherein, when computing the backward values, a reverse of the topological orderings are used for the vertices of the K trellises.

10. The computer system of claim 7, wherein each edge in the K trellises is traversed exactly once when computing the forward values.

11. The computer system of claim 1, wherein each of the K trellises is a directed acyclic graph.

12. The computer system of claim 1, wherein independently constructing the K trellises by modelling the IDS channel as the FSM includes tracking an output pointer pointing to specific nucleotides included in a particular noisy trace, which is included among the K noisy traces, to determine an index of a next output nucleotide that is emitted.

13. The computer system of claim 12, wherein the output pointer determines a position in the particular noisy trace where the next output nucleotide is to be appended.

14. A method (1100) configured to facilitate reductions in cost of encoding and decoding operations used in deoxyribonucleic acid, DNA, data storage systems and to facilitate reducing errors in said encoding and decoding operations while accounting for a code structure used during said encoding and decoding by constructing and using insertion-deletion-substitution, IDS, trellises for multiple traces, said method comprising:

after a data message sequence, which includes a plurality of symbols, has been encoded into a DNA sequence and after the DNA sequence has been synthesized into a plurality of DNA strands, using (1105) a DNA sequencing channel to randomly sample and sequence K DNA strands included among the plurality of DNA strands, wherein:

said sequencing results in a generation of K noisy traces of the DNA sequence, wherein K is a number greater than 1, and
the DNA sequencing channel is modeled as a simplistic zeroth order approximation in a form of an IDS channel;

independently constructing (1110) a corresponding trellis for each respective noisy trace in the K noisy traces such that K trellises are independently constructed, wherein the IDS channel is modeled as a finite state machine, FSM, to construct each of the K trellises;
running (1115) a forward-backward algorithm on each of the K trellises to compute posterior marginal probabilities for vertices included in each of the K trellises;
computing (1120) an estimate of the data message sequence by iterating through the following steps until all estimated symbols forming the estimate of the data message sequence are determined:

for a given symbol that is to be included in the estimate of the data message sequence, computing a current probability-based belief that each of the K trellises has regarding what the given symbol should be such that a plurality of current probability-based beliefs are computed, said current probability-based beliefs being computed based on the posterior marginal probabilities included in each of the K trellises;
selecting a particular symbol as the given symbol based on an aggregation of the plurality of current probability-based beliefs;
identifying certain vertices in each of the K trellises, said certain vertices being identified as a result of the certain vertices disagreeing, based on those certain vertices' corresponding posterior marginal probabilities, with the decision to select the particular symbol as the given symbol;
updating those certain vertices by updating the posterior marginal probabilities for those certain vertices based on the decision;
performing forward passes on the K trellises to update the K trellises based on updating the certain vertices; and
adding the particular symbol to the estimate of the data message sequence.

15. The method of claim 14, wherein a number of symbol mismatches between symbols included in the data message sequence and symbols included in the estimate of the data message sequence is minimized by reducing a Hamming distance between the data message sequence and the estimate of the data message sequence.

**Patentansprüche**

1. Computersystem (1200), das eingerichtet ist zur Erleichterung von Kostensenkungen bei Codier- und Decodiervorgängen, die in Datenspeichersystemen für Desoxyribonukleinsäure, DNA, verwendet werden, und zur Erleichterung der Reduktion von Fehlern bei den Codier- und Decodiervorgängen unter Berücksichtigung einer Codestruktur, die während des Codierens und Decodierens verwendet wird, indem Insertions-Deletions-Substitutions-, IDS-, Trellisen für mehrere Spuren konstruiert und verwendet werden, wobei das Computersystem Folgendes umfasst:

einen oder mehrere Prozessoren (1205); und
eine oder mehrere computerlesbare Hardwarespeichervorrichtungen (1215), die Befehle speichern, die durch den einen oder die mehreren Prozessoren ausführbar sind, um das Computersystem mindestens zu Folgendem zu veranlassen:

nachdem eine Datennachrichtensequenz, die eine Mehrzahl von Symbolen aufweist, in eine DNA-Sequenz codiert wurde und nachdem die DNA-Sequenz in eine Mehrzahl von DNA-Strängen synthetisiert wurde, Verwenden (1105) eines DNA-Sequenzierungskanals, um K DNA-Stränge, die in der Mehrzahl von DNA-Strängen aufgenommen sind, zufällig abzutasten und zu sequenzieren, wobei:

das Sequenzieren zu einer Erzeugung von K verrauschten Spuren der DNA-Sequenz führt, wobei K eine Zahl größer als 1 ist, und
der DNA-Sequenzierungskanal als eine simplistische Näherung nullter Ordnung in Form eines IDS-Kanals modelliert wird;

unabhängiges Konstruieren (1110) eines entsprechenden Trellis für jede jeweilige verrauschte Spur in den K verrauschten Spuren, sodass K Trellisen unabhängig konstruiert werden, wobei der IDS-Kanal als eine endliche Zustandsmaschine, FSM, modelliert wird, um jede der K Trellisen zu konstruieren;
Ausführen (1115) eines Vorwärts-Rückwärts-Algorithmus auf jeder der K Trellisen, um posteriore marginale Wahrscheinlichkeiten für Vertices, die in jeder der K Trellisen aufgenommen sind, zu berechnen;
Berechnen (1120) einer Schätzung der Datennachrichtensequenz durch Iterieren durch die folgenden Schritte, bis alle geschätzten Symbole, die die Schätzung der Datennachrichtensequenz bilden, bestimmt sind:

für ein gegebenes Symbol, das in die Schätzung der Datennachrichtensequenz einzubeziehen ist, Berechnen einer aktuellen wahrscheinlichkeitsbasierten Annahme, die jede der K Trellisen hinsichtlich dessen hat, was das gegebene Symbol sein sollte, sodass eine Mehrzahl von aktuellen wahrscheinlichkeitsbasierten Annahmen berechnet wird, wobei die aktuellen wahrscheinlichkeitsbasierten Annahmen basierend auf den posterioren marginalen Wahrscheinlichkeiten, die in jeder der K Trellisen aufgenommen sind, berechnet werden;
Auswählen eines bestimmten Symbols als das gegebene Symbol basierend auf einer Aggregation der Mehrzahl von aktuellen wahrscheinlichkeitsbasierten Annahmen;
Identifizieren bestimmter Vertices in jeder der K Trellisen, wobei die bestimmten Vertices als ein Ergebnis davon identifiziert werden, dass die bestimmten Vertices nicht übereinstimmen, basierend auf den entsprechenden posterioren marginalen Wahrscheinlichkeiten dieser bestimmten Vertices, mit der Entscheidung, das bestimmte Symbol als das gegebene Symbol auszuwählen;
Aktualisieren dieser bestimmten Vertices durch Aktualisieren der posterioren marginalen Wahrscheinlichkeiten für diese bestimmten Vertices basierend auf der Entscheidung;
Durchführen von Vorwärtsdurchgängen an den K Trellisen, um die K Trellisen basierend auf dem Aktualisieren der bestimmten Vertices zu aktualisieren; und
Hinzufügen des bestimmten Symbols zu der Schätzung der Datennachrichtensequenz.

2. Computersystem nach Anspruch 1, wobei eine Anzahl von Symbolfehlanpassungen zwischen Symbolen, die in der Datennachrichtensequenz aufgenommen sind, und Symbolen, die in der Schätzung der Datennachrichtensequenz aufgenommen sind, durch Reduzieren eines Hamming-Abstands zwischen der Datennachrichtensequenz und der Schätzung der Datennachrichtensequenz minimiert wird.

3. Computersystem nach Anspruch 1, wobei mindestens ein Trellis in den K Trellisen mit einer Dummy-Stufe vorangestellt ist, die einen Vertex aufweist, der strukturiert ist, um Intra-Stufen-Kanten an einer ersten Stufe des mindestens einen Trellis zu verbieten.

4. Computersystem nach Anspruch 1, wobei ein erstes Trellis, das unter den K Trellisen aufgenommen ist, einen ersten Satz von Vertices und Kanten zwischen Paaren von Vertices in dem ersten Satz von Vertices aufweist,

wobei jede Kante in dem ersten Trellis gewichtet ist, und
wobei eine Summe aller Gewichtungen für alle Kanten eines bestimmten Vertex in dem ersten Trellis einen Wert von Eins aufweist.

5. Computersystem nach Anspruch 4, wobei kein Pfad, der mehrere Vertices in dem ersten Trellis überspannt, zwei Kanten aufweist, die zwei Realisierungen derselben beobachtbaren Größe darstellen.

6. Computersystem nach Anspruch 4, wobei das erste Trellis eine gemeinsame Verteilung induziert, so dass das erste Trellis ein Hidden-Markov-Modell ist.

7. Computersystem nach Anspruch 1, wobei das Ausführen des Vorwärts-Rückwärts-Algorithmus Folgendes aufweist:

Modifizieren der K Trellisen, um einen Satz von Beobachtungen zu erklären;
Berechnen von Vorwärtswerten für jeden Vertex in jedem der K Trellisen;
Berechnen von Rückwärtswerten für jeden Vertex in jedem der K Trellisen; und
Berechnen der posterioren marginalen Wahrscheinlichkeiten für die Vertices in jeder der K Trellisen unter Verwendung der Vorwärtswerte und der Rückwärtswerte.

8. Computersystem nach Anspruch 7, wobei das Berechnen der Vorwärtswerte ein Berechnen topologischer Ordnungen für die Vertices der K Trellisen aufweist.

9. Computersystem nach Anspruch 8, wobei beim Berechnen der Rückwärtswerte eine Umkehrung der topologischen Ordnungen für die Vertices der K Trellisen verwendet wird.

10. Computersystem nach Anspruch 7, wobei jede Kante in den K Trellisen beim Berechnen der Vorwärtswerte genau einmal durchlaufen wird.

11. Computersystem nach Anspruch 1, wobei jede der K Trellisen ein gerichteter azyklischer Graph ist.

12. Computersystem nach Anspruch 1, wobei das unabhängige Konstruieren der K Trellisen durch Modellieren des IDS-Kanals als die FSM ein Verfolgen eines Ausgangszeigers aufweist, der auf spezifische Nukleotide zeigt, die in einer bestimmten verrauschten Spur aufgenommen sind, die unter den K verrauschten Spuren aufgenommen ist, um einen Index eines nächsten Ausgangsnukleotids zu bestimmen, das emittiert wird.

13. Computersystem nach Anspruch 12, wobei der Ausgangszeiger eine Position in der bestimmten verrauschten Spur bestimmt, an die das nächste Ausgangsnukleotid angehängt werden soll.

14. Verfahren (1100), das eingerichtet ist zur Erleichterung von Kostensenkungen bei Codier- und Decodiervorgängen, die in Datenspeichersystemen für Desoxyribonukleinsäure, DNA, verwendet werden, und zur Erleichterung der Reduktion von Fehlern bei den Codier- und Decodiervorgängen unter Berücksichtigung einer Codestruktur, die während des Codierens und Decodierens verwendet wird, indem Insertions-Deletions-Substitutions-, IDS-, Trellisen für mehrere Spuren konstruiert und verwendet werden, wobei das Verfahren folgende Schritte umfasst:

nachdem eine Datennachrichtensequenz, die eine Mehrzahl von Symbolen aufweist, in eine DNA-Sequenz codiert wurde und nachdem die DNA-Sequenz in eine Mehrzahl von DNA-Strängen synthetisiert wurde, Verwenden (1105) eines DNA-Sequenzierungskanals, um K DNA-Stränge, die in der Mehrzahl von DNA-Strängen aufgenommen sind, zufällig abzutasten und zu sequenzieren, wobei:

das Sequenzieren zu einer Erzeugung von K verrauschten Spuren der DNA-Sequenz führt, wobei K eine Zahl größer als 1 ist, und

der DNA-Sequenzierungskanal als eine simplistische Näherung nullter Ordnung in Form eines IDS-Kanals modelliert wird;

unabhängiges Konstruieren (1110) eines entsprechenden Trellis für jede jeweilige verrauschte Spur in den K verrauschten Spuren, sodass K Trellisen unabhängig konstruiert werden, wobei der IDS-Kanal als eine endliche Zustandsmaschine, FSM, modelliert wird, um jede der K Trellisen zu konstruieren;

Ausführen (1115) eines Vorwärts-Rückwärts-Algorithmus auf jeder der K Trellisen, um posteriore marginale Wahrscheinlichkeiten für Vertices, die in jeder der K Trellisen aufgenommen sind, zu berechnen;

Berechnen (1120) einer Schätzung der Datennachrichtensequenz durch Iterieren durch die folgenden Schritte, bis alle geschätzten Symbole, die die Schätzung der Datennachrichtensequenz bilden, bestimmt sind:

für ein gegebenes Symbol, das in die Schätzung der Datennachrichtensequenz einzubeziehen ist, Berechnen einer aktuellen wahrscheinlichkeitsbasierten Annahme, die jede der K Trellisen hinsichtlich dessen hat, was das gegebene Symbol sein sollte, sodass eine Mehrzahl von aktuellen wahrscheinlichkeitsbasierten Annahmen berechnet wird, wobei die aktuellen wahrscheinlichkeitsbasierten Annahmen basierend auf den posterioren marginalen Wahrscheinlichkeiten, die in jeder der K Trellisen aufgenommen sind, berechnet werden;

Auswählen eines bestimmten Symbols als das gegebene Symbol basierend auf einer Aggregation der Mehrzahl von aktuellen wahrscheinlichkeitsbasierten Annahmen;

Identifizieren bestimmter Vertices in jeder der K Trellisen, wobei die bestimmten Vertices als ein Ergebnis davon identifiziert werden, dass die bestimmten Vertices nicht übereinstimmen, basierend auf den entsprechenden posterioren marginalen Wahrscheinlichkeiten dieser bestimmten Vertices, mit der Entscheidung, das bestimmte Symbol als das gegebene Symbol auszuwählen;

Aktualisieren dieser bestimmten Vertices durch Aktualisieren der posterioren marginalen Wahrscheinlichkeiten für diese bestimmten Vertices basierend auf der Entscheidung;

Durchführen von Vorwärtsdurchgängen an den K Trellisen, um die K Trellisen basierend auf dem Aktualisieren der bestimmten Vertices zu aktualisieren; und

Hinzufügen des bestimmten Symbols zu der Schätzung der Datennachrichtensequenz.

**15.** Verfahren nach Anspruch 14, wobei eine Anzahl von Symbolfehlanpassungen zwischen Symbolen, die in der Datennachrichtensequenz aufgenommen sind, und Symbolen, die in der Schätzung der Datennachrichtensequenz aufgenommen sind, durch Reduzieren eines Hamming-Abstands zwischen der Datennachrichtensequenz und der Schätzung der Datennachrichtensequenz minimiert wird.

## Revendications

**1.** Système informatique (1200) configuré pour faciliter des réductions de coût d'opérations de codage et de décodage utilisées dans des systèmes de stockage de données d'acide désoxyribonucléique, ADN, et pour faciliter la réduction d'erreurs dans lesdites opérations de codage et de décodage tout en tenant compte d'une structure de code utilisée pendant ledit codage et décodage en construisant et en utilisant des triplets d'insertion-suppression-substitution, IDS, pour de multiples traces, ledit système informatique comprenant :

un ou plusieurs processeurs (1205) ; et

un ou plusieurs dispositifs de stockage matériel lisibles par ordinateur (1215) qui stockent des instructions qui sont exécutables par les un ou plusieurs processeurs pour amener le système informatique à au moins :

après qu'une séquence de message de données, qui inclut une pluralité de symboles, a été codée en une séquence d'ADN et après que la séquence d'ADN a été synthétisée en une pluralité de brins d'ADN, utiliser (1105) un canal de séquençage d'ADN pour échantillonner et séquencer aléatoirement K brins d'ADN inclus parmi la pluralité de brins d'ADN, dans lequel :

ledit séquençage aboutit à une génération de K traces bruyantes de la séquence d'ADN, dans lequel K est un nombre supérieur à 1, et

le canal de séquençage d'ADN est modélisé comme une approximation d'ordre zéro simpliste sous la forme d'un canal IDS ;

construire indépendamment (1110) un treillis correspondant pour chaque trace bruyante respective dans les K traces bruyantes de sorte que K triplets soient construits indépendamment, dans lequel le canal IDS est modélisé comme une machine à états finis, FSM, pour construire chacun des K triplets ;

exécuter (1115) un algorithme avant-arrière sur chacun des K triplets pour calculer des probabilités marginales postérieures pour des sommets inclus dans chacun des K triplets ;

calculer (1120) une estimation de la séquence de message de données en itérant les étapes suivantes jusqu'à ce que tous les symboles estimés formant l'estimation de la séquence de message de données soient déterminés :

pour un symbole donné qui doit être inclus dans l'estimation de la séquence de message de données, calculer une croyance basée sur la probabilité actuelle que chacun des K triplets a concernant ce que le symbole donné devrait être de sorte qu'une pluralité de croyances basées sur la probabilité actuelles soient calculées, lesdites croyances basées sur la probabilité actuelles étant calculées sur la base des probabilités marginales postérieures incluses dans chacun des K triplets ;

sélectionner un symbole particulier comme le symbole donné sur la base d'une agrégation de la pluralité de croyances basées sur la probabilité actuelles ;

identifier certains sommets dans chacun des K triplets, lesdits certains sommets étant identifiés comme un résultat du désaccord des certains sommets, sur la base des probabilités marginales postérieures correspondantes de ces certains sommets, avec la décision de sélectionner le symbole particulier comme le symbole donné ;

mettre à jour ces certains sommets en mettant à jour les probabilités marginales postérieures pour ces certains sommets sur la base de la décision ;

effectuer des passages avant sur les K triplets pour mettre à jour les K triplets sur la base de la mise à jour des certains sommets ; et

ajouter le symbole particulier à l'estimation de la séquence de message de données.

2. Système informatique selon la revendication 1, dans lequel un nombre de désadaptations de symboles entre des symboles inclus dans la séquence de message de données et des symboles inclus dans l'estimation de la séquence de message de données est minimisé en réduisant une distance de Hamming entre la séquence de message de données et l'estimation de la séquence de message de données.

3. Système informatique selon la revendication 1, dans lequel au moins un treillis dans les K triplets est précédé d'un étage factice ayant un sommet structuré pour interdire des arêtes intra-étage à un premier étage de l'au moins un treillis.

4. Système informatique selon la revendication 1, dans lequel un premier treillis inclus parmi les K triplets inclut un premier ensemble de sommets et d'arêtes entre des paires de sommets dans le premier ensemble de sommets,

dans lequel chaque arête dans le premier treillis est pondérée, et

dans lequel une somme de tous les poids pour toutes les arêtes de n'importe quel sommet particulier dans le premier treillis a une valeur de un.

5. Système informatique selon la revendication 4, dans lequel aucun chemin couvrant de multiples sommets dans le premier treillis ne contient deux arêtes correspondant à deux réalisations d'un même observable.

6. Système informatique selon la revendication 4, dans lequel le premier treillis induit une distribution conjointe de sorte que le premier treillis soit un modèle de Markov caché.

7. Système informatique selon la revendication 1, dans lequel l'exécution de l'algorithme avant-arrière inclut :

la modification des K triplets pour expliquer un ensemble d'observations ;

le calcul de valeurs avant pour chaque sommet dans chacun des K triplets ;

le calcul de valeurs arrière pour chaque sommet dans chacun des K triplets ; et

le calcul des probabilités marginales postérieures pour les sommets dans chacun des K triplets en utilisant les valeurs avant et les valeurs arrière.

8. Système informatique selon la revendication 7, dans lequel le calcul des valeurs avant inclut le calcul d'ordres topologiques pour les sommets des K triplets.

9. Système informatique selon la revendication 8, dans lequel, lors du calcul des valeurs arrière, un inverse des ordres topologiques est utilisé pour les sommets des K triplets.

10. Système informatique selon la revendication 7, dans lequel chaque arête dans les K triplets est traversée exactement une fois lors du calcul des valeurs avant.

11. Système informatique selon la revendication 1, dans lequel chacun des K triplets est un graphe acyclique dirigé.

12. Système informatique selon la revendication 1, dans lequel la construction indépendante des K triplets en modélisant le canal IDS comme la FSM inclut le suivi d'un pointeur de sortie pointant vers des nucléotides spécifiques inclus dans une trace bruyante particulière, qui est incluse parmi les K traces bruyantes, pour déterminer un indice d'un nucléotide de sortie suivant qui est émis.

13. Système informatique selon la revendication 12, dans lequel le pointeur de sortie détermine une position dans la trace bruyante particulière où le nucléotide de sortie suivant doit être ajouté.

14. Procédé (1100) configuré pour faciliter des réductions de coût d'opérations de codage et de décodage utilisées dans des systèmes de stockage de données d'acide désoxyribonucléique, ADN, et pour faciliter la réduction d'erreurs dans lesdites opérations de codage et de décodage tout en tenant compte d'une structure de code utilisée pendant ledit codage et décodage en construisant et en utilisant des triplets d'insertion-suppression-substitution, IDS, pour de multiples traces, ledit procédé comprenant :

après qu'une séquence de message de données, qui inclut une pluralité de symboles, a été codée en une séquence d'ADN et après que la séquence d'ADN a été synthétisée en une pluralité de brins d'ADN, utiliser (1105) un canal de séquençage d'ADN pour échantillonner et séquencer aléatoirement K brins d'ADN inclus parmi la pluralité de brins d'ADN, dans lequel :

ledit séquençage aboutit à une génération de K traces bruyantes de la séquence d'ADN, dans lequel K est un nombre supérieur à 1, et
le canal de séquençage d'ADN est modélisé comme une approximation d'ordre zéro simpliste sous la forme d'un canal IDS ;

construire indépendamment (1110) un treillis correspondant pour chaque trace bruyante respective dans les K traces bruyantes de sorte que K triplets soient construits indépendamment, dans lequel le canal IDS est modélisé comme une machine à états finis, FSM, pour construire chacun des K triplets ;
exécuter (1115) un algorithme avant-arrière sur chacun des K triplets pour calculer des probabilités marginales postérieures pour des sommets inclus dans chacun des K triplets ;
calculer (1120) une estimation de la séquence de message de données en itérant les étapes suivantes jusqu'à ce que tous les symboles estimés formant l'estimation de la séquence de message de données soient déterminés :

pour un symbole donné qui doit être inclus dans l'estimation de la séquence de message de données, calculer une croyance basée sur la probabilité actuelle que chacun des K triplets a concernant ce que le symbole donné devrait être de sorte qu'une pluralité de croyances basées sur la probabilité actuelles soient calculées, lesdites croyances basées sur la probabilité actuelles étant calculées sur la base des probabilités marginales postérieures incluses dans chacun des K triplets ;
sélectionner un symbole particulier comme le symbole donné sur la base d'une agrégation de la pluralité de croyances basées sur la probabilité actuelles ;
identifier certains sommets dans chacun des K triplets, lesdits certains sommets étant identifiés comme un résultat du désaccord des certains sommets, sur la base des probabilités marginales postérieures correspondantes de ces certains sommets, avec la décision de sélectionner le symbole particulier comme le symbole donné ;
mettre à jour ces certains sommets en mettant à jour les probabilités marginales postérieures pour ces certains sommets sur la base de la décision ;
effectuer des passages avant sur les K triplets pour mettre à jour les K triplets sur la base de la mise à jour des certains sommets ; et
ajouter le symbole particulier à l'estimation de la séquence de message de données.

15. Procédé selon la revendication 14, dans lequel un nombre de désadaptations de symboles entre des symboles inclus dans la séquence de message de données et des symboles inclus dans l'estimation de la séquence de message de données est minimisé en réduisant une distance de Hamming entre la séquence de message de données et l'estimation de la séquence de message de données.

DNA Data Storage Model
100

IDS Model
100A

Encode
110

Symbol
105B

11001101

Binary Data
105

ACTGATGCA

GCTTACGGA

TTCCGAGTA

Base Sequence
115

Synthesis
120

Synthesized DNA
Strand
125

Sequence
130

aACTtATGCA

GCTTcCGGA

GCaTACGG

CTGATGCA

Base Sequence
135

Decode
140

Estimated
Symbol
145B

11001101

Binary Data
145

M

Message Sequence
105A

X

Encoded DNA
Sequence
115A

$Y^1$
$Y^2$
$Y^3$

Multiple Noisy
Observations
(traces)
135A

$\hat{M}$

Estimate Of The
Message
145A

*Figure 1A*

S ⟶ Substitution

I ⟶ Insertion

D ⟶ Deletion

I  S

aACTtATGCA

Decoded Base
Sequence
155A

ACTGATGCA

Original Base
Sequence
150

D

CTGATGCA

Decoded Base
Sequence
155B

*Figure 1B*

S → Substitution

I → Insertion

D → Deletion

Decoded Base
Sequence
165A

S

↓

GCTTcCGGA

GCTTACGGA

S          D

↓          ↓

GCaTACGG

Original Base
Sequence
160

Decoded Base
Sequence
165B

EP 4 252 351 B1

34

*Figure 1C*

S ⟶ Substitution

I ⟶ Insertion

D ⟶ Deletion

Lost Base Sequence
175

TTCCGAGTA ⟶ ????????

Original Base
Sequence
170

*Figure 1D*

Probabilistic FSM
200

0 / A / 0.6

0 / B / 1.0

1 / B / 1.0

State
205

State
210

0

1

Edge
220

1 / A / 0.3

0 / B / 0.4

Edge Label
230

1 / B / 0.7

Input
215

Output
225

Weight
235

*Figure 2*

Figure 3

$$X_1 X_2 \dots X_i \dots$$

$$Y_1 Y_2 \dots Y_p \dots$$

$$i$$

$$P_i$$

Input Pointer
405

Output Pointer
400

$$P_i = p + 1$$

$$P_{i+1} = p + 1$$

Insertion

Substitution/
Replication

$$P_i = p$$

$$P_{i+1} = p$$

Deletion

**Figure 4**

Thee Input Cycles For A Trellis
500

inp    out    tns    inp    out    tns    inp    out    tns    end

*Figure 5*

EP 4 252 351 B1

Two Input Cycles In The IDS Trellis For One Trace
600

Figure 6

Multi-Trace IDS Trellis
700

**Figure 7**

41

Comparison
800

Figure 8

EP 4 252 351 B1

Figure 9

Trellis
1000

Figure 10

Use A DNA Sequencing Channel To Randomly Sample And Sequence K DNA Strands Included Among The Plurality Of DNA Strands ⟿ 1105

Independently Construct A Corresponding Trellis For Each Respective Noisy Trace In The K Noisy Traces Such That K Trellises Are Independently Constructed ⟿ 1110

Run A Forward-backward Algorithm On Each Of The K Trellises To Compute Posterior Marginal Probabilities For Vertices Included In Each Of The K Trellises ⟿ 1115

Compute An Estimate Of The Data Message Sequence ⟿ 1120

*Figure 11A*

For A Given Symbol That Is To Be Included In The Estimate Of The Data Message Sequence, Compute A Current Probability-based Belief That Each Of The K Trellises Has Regarding What The Given Symbol Should Be Such That A Plurality Of Current Probability-based Beliefs Are Computed, Said Current Probability-based Beliefs Being Computed Based On The Posterior Marginal Probabilities Included In Each Of The K Trellises ⟿ 1125

Select A Particular Symbol As The Given Symbol Based On An Aggregation Of The Plurality Of Current Probability-based Beliefs ⟿ 1130

Identify Certain Vertices In Each Of The K Trellises, Said Certain Vertices Being Identified As A Result Of The Certain Vertices Disagreeing, Based On Those Certain Vertices' Corresponding Posterior Marginal Probabilities, With The Decision To Select The Particular Symbol As The Given Symbol ⟿ 1135

Update Those Certain Vertices By Updating The Posterior Marginal Probabilities For Those Certain Vertices Based On The Decision ⟿ 1140

Perform Forward Passes On The K Trellises To Update The K Trellises Based On Updating The Certain Vertices ⟿ 1145

Add The Particular Symbol To The Estimate Of The Data Message Sequence ⟿ 1150

*Figure 11B*

Computer System
1200

Processor(s)
1205

I/O
1210

Storage
1215

Code
1220

Network
1225

*Figure 12*

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LENZ ANDREAS et al.** Concatenated Codes for Recovery From Multiple Reads of DNA Sequences. *2020 IEEE Information Theory Workshop (ITW)*, 29 October 2020, 1-5, 8